# EUROPEAN PATENT SPECIFICATION

(11) **EP 2 045 322 B1**
(45) Date of publication and mention of the grant of the patent: **01.07.2015**
(21) Application number: 08006554.3
(22) Date of filing: 14.07.1998
(51) Int. Cl.: C12N 15/00, C12N 15/12, C07K 14/495, C12N 5/10, C12Q 1/68, A01K 67/027, A61K 48/00

(54) **Double-muscling in mammals**
Doppelmuskelbildung bei Säugetieren
Musculation double chez les mammifères

(30) Priority: 14.07.1997 US 891789; 15.01.1998 US 7761
(43) Date of publication of application: 08.04.2009
(62) Divisional of application: 98935228.1
(73) Proprietor: Université de Liège, 4031 Angleur (BE)
(72) Inventor: Grobet, Luc, 4130 Esneux (BE); Georges, Michel, 4161 Villers-aux-Tours (BE); Poncelet, Dominique, 3700 Tongeren (BE)
(74) Representative: Vossius & Partner Patentanwälte Rechtsanwälte mbB

(56) References cited:
- WO-A-94/21681
- MCPHERRON, LAWLER AND LEE: "Regulation of skeletal muscle mass in mice by a new TGF-beta superfamily member." NATURE, vol. 387, 1 May 1997 (1997-05-01), pages 83-90, XP002085797
- CHARLIER ET AL.: "The mh gene causing double-muscling in cattle maps to the bovine chromosome 2." MAMMALIAN GENOME, vol. 6, no. 11, 1995, pages 788-792, XP002085798
- GROBET ET AL.: "Molecular definition of an allelic series of mutations disrupting the myostatin function and causing double-muscling in cattle" MAMM. GENOME, vol. 9, no. 3, March 1998 (1998-03), pages 210-213, XP002085799
- GROBET ET AL.: "A deletion in the bovine myostatin gene causes the double-muscled phenotype in cattle." NATURE GENETICS, vol. 17, no. 1, September 1997 (1997-09), pages 71-74, XP002085800
- MCPHERRON AND LEE: "Double muscling in cattle due to mutations in the myostatin gene" PROC. NATL. ACAD. SCI. USA, vol. 94, no. 23, 11 November 1997 (1997-11-11), pages 12457-12461, XP002085801
- KAMBADUR ET AL.: "Mutations in myostatin (GDF8) in double-muscled Belgian Blue and Piedmontese cattle." GENOME RESEARCH, vol. 7, no. 9, September 1997 (1997-09), pages 910-916, XP002085802
- WESTHUSIN. M.: "For mighty mice to mighty cows" NATURE GENETICS, vol. 17, no. 1, September 1997 (1997-09), pages 71-74, XP002086548
- GEORGES AND ANDERSSON: "Livestock genomics comes of age" GENOME RESEARCH, vol. 6, 1996, pages 907-921, XP002085805
- KAPPES ET AL.: "A second-generation linkage map of the bovine genome" GENOME RESEARCH, vol. 7, 1997, pages 235-249, XP002085806

### SEQUENCE LISTING

### SEQUENCE LISTING

<110> UNIVERSITY OF LIEGE
<120> DOUBLE-MUSCLING IN MAMMALS
<130> C2087 EP/1 S3
<150> EP 98 93 5228.1
   <151> 1998-07-14
<150> US 19970891789
   <151> 1997-07-14
<150> US 19980007761
   <151> 1998-01-15
<160> 54
<170> PatentIn ver. 2.0
(2) INFORMATION FOR SEQ ID NO:1
   (i) SEQUENCE CHARACTERISTICS:
      (A) LENGTH: 1196 base pairs
      (B) TYPE: nucleic acid
      (C) STRANDEDNESS: single
      (D) TOPOLOGY: linear
   (xi) SEQUENCE DESCRIPTION: SEQ ID NO:1
(2) INFORMATION FOR SEQ ID NO:2
   (i) SEQUENCE CHARACTERISTICS:
      (A) LENGTH: 375 amino acids
      (B) TYPE: amino acid
      (C) STRANDEDNESS: single
      (D) TOPOLOGY: linear
   (Xi) SEQUENCE DESCRIPTION: SEQ ID NO:2
   Met Gln Lys Leu Gln Ile Ser Val Tyr Ile Tyr Leu Phe Met Leu Ile
   1               5                   10                  15
   Val Ala Gly Pro Val Asp Leu Asn Glu Asn Ser Glu Gln Lys Glu Asn
               20                  25                  30
   Val Glu Lys Glu Gly Leu Cys Asn Ala Cys Leu Trp Arg Glu Asn Thr
           35                  40                  45
   Thr Ser Ser Arg Leu Glu Ala Ile Lys Ile Gln Ile Leu Ser Lys Leu
       50                  55                  60
   Arg Leu Glu Thr Ala Pro Asn Ile Ser Lys Asp Ala Ile Arg Gln Leu
   65                  70                  75                  80
   Leu Pro Lys Ala Pro Pro Leu Leu Glu Leu Ile Asp Gln Phe Asp Val
                   85                  90                  95
   Gln Arg Asp Ala Ser Ser Asp Gly Ser Leu Glu Asp Asp Asp Tyr His
               100                 105                 110
   Ala Arg Thr Glu Thr Val Ile Thr Met Pro Thr Glu Ser Asp Leu Leu
           115                 120                 125
   Thr Gln Val Glu Gly Lys Pro Lys Cys Cys Phe Phe Lys Phe Ser Ser
       130                 135                 140
   Lys Ile Gln Tyr Asn Lys Leu Val Lys Ala Gln Leu Trp Ile Tyr Leu
   145                 150                 155                 160
   Arg Pro Val Lys Thr Pro Ala Thr Val Phe Val Gln Ile Leu Arg Leu
                   165                 170                 175
   Ile Lys Pro Met Lys Asp Gly Thr Arg Tyr Thr Gly Ile Arg Ser Leu
               180                 185                 190
   Lys Leu Asp Met Asn Pro Gly Thr Gly Ile Trp Gln Ser Ile Asp Val
           195                 200                 205
   Lys Thr Val Leu Gln Asn Trp Leu Lys Gln Pro Glu Ser Asn Leu Gly
       210                 215                 220
   Ile Glu Ile Lys Ala Leu Asp Glu Asn Gly His Asp Leu Ala Val Thr
   225                 230                 235                 240
   Phe Pro Glu Pro Gly Glu Asp Gly Leu Thr Pro Phe Leu Glu Val Lys
                   245                 250                 255
   Val Thr Asp Thr Pro Lys Arg Ser Arg Arg Asp Phe Gly Leu Asp Cys
               260                 265                 270
   Asp Glu His Ser Thr Glu Ser Arg Cys Cys Arg Tyr Pro Leu Thr Val
           275                 280                 285
   Asp Phe Glu Ala Phe Gly Trp Asp Trp Ile Ile Ala Pro Lys Arg Tyr
       290                 295                 300
   Lys Ala Asn Tyr Cys Ser Gly Glu Cys Glu Phe Val Phe Leu Gln Lys
   305                 310                 315                 320
   Tyr Pro His Thr His Leu Val His Gln Ala Asn Pro Arg Gly Ser Ala
   325 330 335
   Gly Pro Cys Cys Thr Pro Thr Lys Met Ser Pro Ile Asn Met Leu Tyr
   340 345 350
   Phe Asn Gly Glu Gly Gln Ile Ile Tyr Gly Lys Ile Pro Ala Met Val
   355 360 365
   Val Asp Arg Cys Gly Cys Ser
   370 375
(2) INFORMATION FOR SEQ ID NO:3
   (i) SEQUENCE CHARACTERISTICS:
      (A) LENGTH: 1240 base pairs
      (B) TYPE: nucleic acid
      (C) STRANDEDNESS: single
      (D) TOPOLOGY: linear
   (xi) SEQUENCE DESCRIPTION: SEQ ID NO:3
   AGGAAAAAGT AAGAACAAGG GAAAAGATTG TATTGATTTT AAAACC ATG CAA AAA      55
                                                     Met Gln Lys
                                                      1
   CTG CAA ATC TCT GTT TAT ATT TAC CTA TTT ATG CTC ATT GTT GCT GGC    103
   Leu Gln Ile Ser Val Tyr Ile Tyr Leu Phe Met Leu Ile Val Ala Gly
        5                  10                  15
   CCA GTG GAT CTG AAT GAG AAC AGC GAG CAG AAG GAA AAT GTG GAA AAA    151
   Pro Val Asp Leu Asn Glu Asn Ser Glu Gln Lys Glu Asn Val Glu Lys
   20                  25                  30                  35
   GAG GGG CTG TGT AAT GCA TGT TTG TGG AGG GAA AAC ACT ACA TCC TCA    199
   Glu Gly Leu Cys Asn Ala Cys Leu Trp Arg Glu Asn Thr Thr Ser Ser
                   40                  45                  50
   AGA CTA GAA GCC ATA AAA ATC CAA ATC CTC AGT AAA CTT CGC CTG GAA    247
   Arg Leu Glu Ala Ile Lys Ile Gln Ile Leu Ser Lys Leu Arg Leu Glu
               55                  60                  65
   ACA GCT CCT AAC ATC AGC AAA GAT GCT ATC AGA CAA CTT TTG CCC AAG    295
   Thr Ala Pro Asn Ile Ser Lys Asp Ala Ile Arg Gln Leu Leu Pro Lys
           70                  75                  80
   GCT CCT CCA CTC CTG GAA CTG ATT GAT CAG TTC GAT GTC CAG AGA GAT    343
   Ala Pro Pro Leu Leu Glu Leu Ile Asp Gln Phe Asp Val Gln Arg Asp
       85                  90                  95
   GCC AGC AGT GAC GGC TCC TTG GAA GAC GAT GAC TAC CAC GCC AGG ACG    391
   Ala Ser Ser Asp Gly Ser Leu Glu Asp Asp Asp Tyr His Ala Arg Thr
   100                 105                 110                 115
   GAA ACG GTC ATT ACC ATG CCC ACG GAG TCT GAT CTT CTA ACG CAA GTG    439
   Glu Thr Val Ile Thr Met Pro Thr Glu Ser Asp Leu Leu Thr Gln Val
                   120                 125                 130
   GAA GGA AAA CCC AAA TGT TGC TTC TTT AAA TTT AGC TCT AAG ATA CAA    487
   Glu Gly Lys Pro Lys Cys Cys Phe Phe Lys Phe Ser Ser Lys Ile Gln
               135                 140                 145
   TAC AAT AAA CTA GTA AAG GCC CAA CTG TGG ATA TAT CTG AGG CCT GTC    535
   Tyr Asn Lys Leu Val Lys Ala Gln Leu Trp Ile Tyr Leu Arg Pro Val
           150                 155                 160
   AAG ACT CCT GCG ACA GTG TTT GTG CAA ATC CTC AGA CTC ATC AAA CCC    583
   Lys Thr Pro Ala Thr Val Phe Val Gln Ile Leu Arg Leu Ile Lys Pro
       165                 170                 175
   ATG AAA GAC GGT ACA AGG TAT ACT GGA ATC CGA TCT CTG AAA CTT GAC      631
   Met Lys Asp Gly Thr Arg Tyr Thr Gly Ile Arg Ser Leu Lys Leu Asp
   180                 185                 190                 195
   ATG AAC CCA GGC ACT GGT ATT TGG CAG AGC ATT GAT GTG AAG ACA GTG      679
   Met Asn Pro Gly Thr Gly Ile Trp Gln Ser Ile Asp Val Lys Thr Val
                   200                 205                 210
   TTG CAG AAC TGG CTC AAA CAA CCT GAA TCC AAC TTA GGC ATT GAA ATC      727
   Leu Gln Asn Trp Leu Lys Gln Pro Glu Ser Asn Leu Gly Ile Glu Ile
               215                 220                 225
   AAA GCT TTA GAT GAG AAT GGC CAT GAT CTT GCT GTA ACC TTC CCA GAA      775
   Lys Ala Leu Asp Glu Asn Gly His Asp Leu Ala Val Thr Phe Pro Glu
       230                 235                 240
   CCA GGA GAA GAT GGA CTG ACT CCT TTT TTA GAA GTC AAG GTA ACA GAC      823
   Pro Gly Glu Asp Gly Leu Thr Pro Phe Leu Glu Val Lys Val Thr Asp
       245                 250                 255
   ACA CCA AAA AGA TCT AGG AGA GAT TTT GGG CTT GAT TGT GAC AGA ATC      871
   Thr Pro Lys Arg Ser Arg Arg Asp Phe Gly Leu Asp Cys Asp Arg Ile
   260                 265                 270                 275
   TCG ATG CTG TCG TTA CCC TCT AAC TGT GGA TTT TGAAGCTTTT               914
   Ser Met Leu Ser Leu Pro Ser Asn Cys Gly Phe
                   280                 285
   GGATGGGATT GGATTATTGC ACCTAAAAGA TATAAGGCCA ATTACTGCTC TGGAGAATGT     974
   GAATTTGTAT TTTTGCAAAA GTATCCTCAT ACCCATCTTG TGCACCAAGC AAACCCCAGA    1034
   GGTTCAGCCG GCCCCTGCTG TACTCCTACA AAGATGTCTC CAATTAATAT GCTATATTTT    1094
   AATGGCGAAG GACAAATAAT ATACGGGAAG ATTCCAGCCA TGGTAGTAAA TCGCTGTGGG    1154
   TGTTCATGAG GTCTATATTT GGTTCATAGC TTCCTCAAAC ATGGAAGGTC TTCCCCTCAA    1214
   CAATTTTGAA ACTGTTGAAA TTATGT                                         1240
(2) INFORMATION FOR SEQ ID NO:4
   (i) SEQUENCE CHARACTERISTICS:
      (A) LENGTH: 286 amino acids
      (B) TYPE: amino acid
      (C) STRANDEDNESS: single
      (D) TOPOLOGY: linear
   (xi) SEQUENCE DESCRIPTION: SEQ ID NO:4
   Met Gln Lys Leu Gln Ile Ser Val Tyr Ile Tyr Leu Phe Met Leu Ile
    1               5                  10                  15
   Val Ala Gly Pro Val Asp Leu Asn Glu Asn Ser Glu Gln Lys Glu Asn
               20                  25                  30
   Val Glu Lys Glu Gly Leu Cys Asn Ala Cys Leu Trp Arg Glu Asn Thr
           35                  40                  45
   Thr Ser Ser Arg Leu Glu Ala Ile Lys Ile Gln Ile Leu Ser Lys Leu
       50                  55                  60
   Arg Leu Glu Thr Ala Pro Asn Ile Ser Lys Asp Ala Ile Arg Gln Leu
   65                  70                  75                  80
   Leu Pro Lys Ala Pro Pro Leu Leu Glu Leu Ile Asp Gln Phe Asp Val
                   85                  90                  95
   Gln Arg Asp Ala Ser Ser Asp Gly Ser Leu Glu Asp Asp Asp Tyr His
               100                 105                 110
   Ala Arg Thr Glu Thr Val Ile Thr Met Pro Thr Glu Ser Asp Leu Leu
           115                 120                 125
   Thr Gln Val Glu Gly Lys Pro Lys Cys Cys Phe Phe Lys Phe Ser Ser
       130                 135                 140
   Lys Ile Gln Tyr Asn Lys Leu Val Lys Ala Gln Leu Trp Ile Tyr Leu
   145                 150                 155                 160
   Arg Pro Val Lys Thr Pro Ala Thr Val Phe Val Gln Ile Leu Arg Leu
                   165                 170                 175
   Ile Lys Pro Met Lys Asp Gly Thr Arg Tyr Thr Gly Ile Arg Ser Leu
               180                 185                 190
   Lys Leu Asp Met Asn Pro Gly Thr Gly Ile Trp Gln Ser Ile Asp Val
           195                 200                 205
   Lys Thr Val Leu Gln Asn Trp Leu Lys Gln Pro Glu Ser Asn Leu Gly
       210                 215                 220
   Ile Glu Ile Lys Ala Leu Asp Glu Asn Gly His Asp Leu Ala Val Thr
   225                 230                 235                 240
   Phe Pro Glu Pro Gly Glu Asp Gly Leu Thr Pro Phe Leu Glu Val Lys
                   245                 250                 255
   Val Thr Asp Thr Pro Lys Arg Ser Arg Arg Asp Phe Gly Leu Asp Cys
               260                 265                 270
   Asp Arg Ile Ser Met Leu Ser Leu Pro Ser Asn Cys Gly Phe
           275                 280                 285
(2) INFORMATION FOR SEQ ID NO:5
   (i) SEQUENCE CHARACTERISTICS:
      (A) LENGTH: 2676 base pairs
      (B) TYPE: nucleic acid
      (C) STRANDEDNESS: single
      (D) TOPOLOGY: linear
   (xi) SEQUENCE DESCRIPTION: SEQ ID NO:5
   GTCTCTCGGA CGGTACATGC ACTAATATTT CACTTGGCAT TACTCAAAAG CAAAAAGAAG        60
   AAATAAGAAC AAGCGAAAAA AAAAGATTGT GCTGATTTTT AAA ATG ATG CAA AAA         115
                                                   Met Met Gln Lys
                                                   1
   CTG CAA ATG TAT GTT TAT ATT TAC CTC TTC ATG CTG ATT GCT GCT GGC        163
   Leu Gln Met Tyr Val Tyr Ile Tyr Leu Phe Met Leu Ile Ala Ala Gly
   5                   10                  15                  20
   CCA GTG GAT CTA AAT GAG GGC AGT GAG AGA GAA GAA AAT GTG GAA AAA        211
   Pro Val Asp Leu Asn Glu Gly Ser Glu Arg Glu Glu Asn Val Glu Lys
                   25                  30                  35
   GAG GGG CTG TGT AAT GCA TGT GCG TGG AGA CAA AAC ACG AGG TAC TCC        259
   Glu Gly Leu Cys Asn Ala Cys Ala Trp Arg Gln Asn Thr Arg Tyr Ser
               40                  45                  50
   AGA ATA GAA GCC ATA AAA ATT CAA ATC CTC AGT AAG CTG CGC CTG GAA        307
   Arg Ile Glu Ala Ile Lys Ile Gln Ile Leu Ser Lys Leu Arg Leu Glu
           55                  60                  65
   ACA GCT CCT AAC ATC AGC AAA GAT GCT ATA AGA CAA CTT CTG CCA AGA      355
   Thr Ala Pro Asn Ile Ser Lys Asp Ala Ile Arg Gln Leu Leu Pro Arg
       70                  75                  80
   GCG CCT CCA CTC CGG GAA CTG ATC GAT CAG TAC GAC GTC CAG AGG GAT      403
   Ala Pro Pro Leu Arg Glu Leu Ile Asp Gln Tyr Asp Val Gln Arg Asp
   85                  90                  95                  100
   GAC AGC AGT GAT GGC TCT TTG GAA GAT GAC GAT TAT CAC GCT ACC ACG      451
   Asp Ser Ser Asp Gly Ser Leu Glu Asp Asp Asp Tyr His Ala Thr Thr
                   105                 110                 115
   GAA ACA ATC ATT ACC ATG CCT ACA GAG TCT GAC TTT CTA ATG CAA GCG      499
   Glu Thr Ile Ile Thr Met Pro Thr Glu Ser Asp Phe Leu Met Gln Ala
               120                 125                 130
   GAT GGC AAG CCC AAA TGT TGC TTT TTT AAA TTT AGC TCT AAA ATA CAG      547
   Asp Gly Lys Pro Lys Cys Cys Phe Phe Lys Phe Ser Ser Lys Ile Gln
           135                 140                 145
   TAC AAC AAA GTA GTA AAA GCC CAA CTG TGG ATA TAT CTC AGA CCC GTC      595
   Tyr Asn Lys Val Val Lys Ala Gln Leu Trp Ile Tyr Leu Arg Pro Val
       150                 155                 160
   AAG ACT CCT ACA ACA GTG TTT GTG CAA ATC CTG AGA CTC ATC AAA CCC      643
   Lys Thr Pro Thr Thr Val Phe Val Gln Ile Leu Arg Leu Ile Lys Pro
   165                 170                 175                 180
   ATG AAA GAC GGT ACA AGG TAT ACT GGA ATC CGA TCT CTG AAA CTT GAC      691
   Met Lys Asp Gly Thr Arg Tyr Thr Gly Ile Arg Ser Leu Lys Leu Asp
                   185                 190                 195
   ATG AGC CCA GGC ACT GGT ATT TGG CAG AGT ATT GAT GTG AAG ACA GTG      739
   Met Ser Pro Gly Thr Gly Ile Trp Gln Ser Ile Asp Val Lys Thr Val
               200                 205                 210
   TTG CAA AAT TGG CTC AAA CAG CCT GAA TCC AAC TTA GGC ATT GAA ATC      787
   Leu Gln Asn Trp Leu Lys Gln Pro Glu Ser Asn Leu Gly Ile Glu Ile
           215                 220                 225
   AAA GCT TTG GAT GAG AAT GGC CAT GAT CTT GCT GTA ACC TTC CCA GGA      835
   Lys Ala Leu Asp Glu Asn Gly His Asp Leu Ala Val Thr Phe Pro Gly
       230                 235                 240
   CCA GGA GAA GAT GGG CTG AAT CCC TTT TTA GAA GTC AAG GTG ACA GAC      883
   Pro Gly Glu Asp Gly Leu Asn Pro Phe Leu Glu Val Lys Val Thr Asp
   245                 250                 255                 260
   ACA CCC AAG AGG TCC CGG AGA GAC TTT GGG CTT GAC TGC GAT GAG CAC      931
   Thr Pro Lys Arg Ser Arg Arg Asp Phe Gly Leu Asp Cys Asp Glu His
                   265                 270                 275
   TCC ACG GAA TCC CGG TGC TGC CGC TAC CCC CTC ACG GTC GAT TTT GAA      979
   Ser Thr Glu Ser Arg Cys Cys Arg Tyr Pro Leu Thr Val Asp Phe Glu
               280                 285                 290
   GCC TTT GGA TGG GAC TGG ATT ATC GCA CCC AAA AGA TAT AAG GCC AAT     1027
   Ala Phe Gly Trp Asp Trp Ile Ile Ala Pro Lys Arg Tyr Lys Ala Asn
           295                 300                 305
   TAC TGC TCA GGA GAG TGT GAA TTT GTG TTT TTA CAA AAA TAT CCG CAT     1075
   Tyr Cys Ser Gly Glu Cys Glu Phe Val Phe Leu Gln Lys Tyr Pro His
       310                 315                 320
   ACT CAT CTT GTG CAC CAA GCA AAC CCC AGA GGC TCA GCA GGC CCT TGC     1123
   Thr His Leu Val His Gln Ala Asn Pro Arg Gly Ser Ala Gly Pro Cys
   325                 330                 335                 340
   TGC ACT CCG ACA AAA ATG TCT CCC ATT AAT ATG CTA TAT TTT AAT GGC       1171
   Cys Thr Pro Thr Lys Met Ser Pro Ile Asn Met Leu Tyr Phe Asn Gly
                   345                 350                 355
   AAA GAA CAA ATA ATA TAT GGC AAA ATT CCA GCC ATG GTA GTA GAC CGC       1219
   Lys Glu Gln Ile Ile Tyr Gly Lys Ile Pro Ala Met Val Val Asp Arg
               360                 365                 370
   TGT GGG TGC TCA TGAGCTTTGC ATTAGGTTAG AAACTTCCCA AGTCATGGAA           1271
   Cys Gly Cys Ser
           375
   GGTCTTCCCC TCAATTTCGA AACTGTGAAT TCAAGCACCA CAGGCTGTAG GCCTTGAGTA     1331
   TGCTCTACTA ACGTAAGCAC AAGCTACAGT GTATGAACTA AAAGAGAGAA TAGATGCAAT     1391
   GGTTGGCATT CAACCACCAA AATAAACCAT ACTATAGGAT GTTGTATGAT TTCCAGAGTT     1451
   TTTGAAATAG ATGGAGATCA AATTACATTT ATGTCCATAT ATGTATATTA CAACTACAAT     1511
   CTAGGCAAGG AAGTGAGAGC ACATCTTGTG GTCTGCTGAG TTAGGAGGGT ATGATTAAAA     1571
   GGTAAAGTCT TATTTCCTAA CAGTTTCACT TAATATTTAC AGAACAATCT ATATGTAGCC     1631
   TTTGTAAAGT GTAGGATTGT TATCATTTAA AAACATCATG TACACTTATA TTTGTATTGT     1691
   ATACTTGGTA AGATAAAATT CCACAAAGTA GGAATGGGGC CTCACATACA CATTGCCATT     1751
   CCTATTATAA TTGGACAATC CACCACGGTG CTAATGCAGT GCTCAATGGC TCCTACTGGA     1811
   CCTCTCGATA GAACACTCTA CAAAGTACGA GTCTCTCTCT CCCTTCCAGG TGCATCTCCA     1871
   CACACACAGC ACTAAGTGTT CAATGCATTT TCTTTAAGGA AAGAAGAATC TTTTTTTCTA     1931
   GAGGTCAACT TTCAGTCAAC TCTAGCACAG CGGGAGTGAC TGCTGCATCT TAAAAGGCAG     1991
   CCAAACAGTA TTCATTTTTT AATCTAAATT TCAAAATCAC TGTCTGCCTT TATCACATGG     2051
   CAATTTTGTG GTAAAATAAT GGAAATGACT GGTTCTATCA ATATTGTATA AAAGACTCTG     2111
   AAACAATTAC ATTTATATAA TATGTATACA ATATTGTTTT GTAAATAAGT GTCTCCTTTT     2171
   ATATTTACTT TGGTATATTT TTACACTAAT GAAATTTCAA ATCATTAAAG TACAAAGACA     2231
   TGTCATGTAT CACAAAAAAG GTGACTGCTT CTATTTCAGA GTGAATTAGC AGATTCAATA     2291
   GTGGTCTTAA AACTCTGTAT GTTAAGATTA GAAGGTTATA TTACAATCAA TTTATGTATT     2351
   TTTTACATTA TCAACTTATG GTTTCATGGT GGCTGTATCT ATGAATGTGG CTCCCAGTCA     2411
   AATTTCAATG CCCCACCATT TTAAAAATTA CAAGCATTAC TAAACATACC AACATGTATC     2471
   TAAAGAAATA CAAATATGGT ATCTCAATAA CAGCTACTTT TTTATTTTAT AATTTGACAA     2531
   TGAATACATT TCTTTTATTT ACTTCAGTTT TATAAATTGG AACTTTGTTT ATCAAATGTA     2591
   TTGTACTCAT AGCTAAATGA AATTATTTCT TACATAAAAA TGTGTAGAAA CTATAAATTA     2651
   AAGTGTTTTC ACATTTTTGA AAGGC                                           2676
(2) INFORMATION FOR SEQ ID NO:6
   (i) SEQUENCE CHARACTERISTICS:
      (A) LENGTH: 376 amino acids
      (B) TYPE: amino acid
      (C) STRANDEDNESS: single
      (D) TOPOLOGY: linear
   (xi) SEQUENCE DESCRIPTION: SEQ ID NO:6
   Met Met Gln Lys Leu Gln Met Tyr Val Tyr Ile Tyr Leu Phe Met Leu
    1               5                  10                  15
   Ile Ala Ala Gly Pro Val Asp Leu Asn Glu Gly Ser Glu Arg Glu Glu
               20                  25                  30
   Asn Val Glu Lys Glu Gly Leu Cys Asn Ala Cys Ala Trp Arg Gln Asn
           35                  40                  45
   Thr Arg Tyr Ser Arg Ile Glu Ala Ile Lys Ile Gln Ile Leu Ser Lys
       50                  55                  60
   Leu Arg Leu Glu Thr Ala Pro Asn Ile Ser Lys Asp Ala Ile Arg Gln
   65                  70                  75                  80
   Leu Leu Pro Arg Ala Pro Pro Leu Arg Glu Leu Ile Asp Gln Tyr Asp
                   85                  90                  95
   Val Gln Arg Asp Asp Ser Ser Asp Gly Ser Leu Glu Asp Asp Asp Tyr
               100                 105                 110
   His Ala Thr Thr Glu Thr Ile Ile Thr Met Pro Thr Glu Ser Asp Phe
           115                 120                 125
   Leu Met Gln Ala Asp Gly Lys Pro Lys Cys Cys Phe Phe Lys Phe Ser
       130                 135                 140
   Ser Lys Ile Gln Tyr Asn Lys Val Val Lys Ala Gln Leu Trp Ile Tyr
   145                 150                 155                 160
   Leu Arg Pro Val Lys Thr Pro Thr Thr Val Phe Val Gln Ile Leu Arg
                   165                 170                 175
   Leu Ile Lys Pro Met Lys Asp Gly Thr Arg Tyr Thr Gly Ile Arg Ser
               180                 185                 190
   Leu Lys Leu Asp Met Ser Pro Gly Thr Gly Ile Trp Gln Ser Ile Asp
           195                 200                 205
   Val Lys Thr Val Leu Gln Asn Trp Leu Lys Gln Pro Glu Ser Asn Leu
       210                 215                 220
   Gly Ile Glu Ile Lys Ala Leu Asp Glu Asn Gly His Asp Leu Ala Val
   225                 230                 235                 240
   Thr Phe Pro Gly Pro Gly Glu Asp Gly Leu Asn Pro Phe Leu Glu Val
                   245                 250                 255
   Lys Val Thr Asp Thr Pro Lys Arg Ser Arg Arg Asp Phe Gly Leu Asp
               260                 265                 270
   Cys Asp Glu His Ser Thr Glu Ser Arg Cys Cys Arg Tyr Pro Leu Thr
           275                 280                 285
   Val Asp Phe Glu Ala Phe Gly Trp Asp Trp Ile Ile Ala Pro Lys Arg
       290                 295                 300
   Tyr Lys Ala Asn Tyr Cys Ser Gly Glu Cys Glu Phe Val Phe Leu Gln
   305                 310                 315                 320
   Lys Tyr Pro His Thr His Leu Val His Gln Ala Asn Pro Arg Gly Ser
                   325                 330                 335
   Ala Gly Pro Cys Cys Thr Pro Thr Lys Met Ser Pro Ile Asn Met Leu
               340                 345                 350
   Tyr Phe Asn Gly Lys Glu Gln Ile Ile Tyr Gly Lys Ile Pro Ala Met
           355                 360                 365
   Val Val Asp Arg Cys Gly Cys Ser
       370                 375
(2) INFORMATION FOR SEQ ID NO:7
   (i) SEQUENCE CHARACTERISTICS:
      (A) LENGTH: 2215 base pairs
      (B) TYPE: nucleic acid
      (C) STRANDEDNESS: single
      (D) TOPOLOGY: linear
   (xi) SEQUENCE DESCRIPTION: SEQ ID NO:7
   NGATTTTCTA ATGCAAGTGG ATGGAAAACC CAAATGTTGC TTCTTTAAAT TTAGCTCTAA 60
   AATACAATAC AATAAAGTAG TAAAGGCCCA ACTATCCATA TATTTGAGAC CCGTCGAGAC 120
   TCCTACAACA GTGTTTGTGC AAATCCTGAG ACTCATCAAA CCTATGAAAG ACGGTACAAG 180
   GTATCTGGAA TCCGATCTCT GAAACTTGAC ATGAACCCAG GCACTGGTAT TTGGGCAGAN 240
   ATTGATGTGA AGACACTGTT GCAAAATTGG CTCAAACAAC CTGAATCCAA CTTAGGCATT 300
   GAAATAAAAG CTTTACATGA GAATGGTCAT GATCTTGCTG TAACCTTCCC AGGACCAGGA 360
   AGAAGATGGG CTGAATCCCT TTTTTAAGAA GGTCAAGGTA ACAGACACAC CAAAAAGATT 420
   CCAGAAGGGA TTTTGGGTCT TGACTGGTGA TGAGCACTCA ACAGAATCAC GATCCTGTCG 480
   TTACCCCCTA ACTGGTGGAT TTTGAAGCCT TTGGGATGGG ATTGGATATC GNNNNNNNNN 540
   NNNNNNNNNN NNNNNNNNNN NNNNNNNNNN NNNNNNNNNN NNNNNNNNNN NNNNNNNNNN 600
   NNNNNNNNNN NNNNNNNNNN NNNNNNNNNN NNNNNNNNNN NNNNNNNNNN NNNNNNNNNN 660
   NNNNNNNNNN NNNNNNNNNN NNNNNNNNNN NNNNNNNNNN NNNNNNNNNN NNNNNNNNNN 720
   NNNNNNNNNN NAGCGATGGT AGTAGACCGC TGTGGGTGCT CAGCGATGGT AGTAGACCGC 780
   TGTGGGTGCT CTTTTCAAGC TGTGAAATTA AGTACCACAG GCTATAGGCC TAGAGTATGC 840
   TACAGTCACT TAAGCATAAG CTACAGTATG TAAACTAAAA GGGGGAANGG GAATATATGC 900
   AATGGTTGGC ATTTAACCAT CCAAACAAAT CATACCAGAA AGTTTTATGA TTTCCANAGT 960
   TTTTTNAGGC NAGAAAGGAG GAGTCAAANT TTCANTCTTA TGGTNNNNNN NNNNNNNNNN 1020
   NNNNNNNNNN NNNNNNNNNN NNNNNNNNNN NNNNNNNNNN NNNNNNNNNN NNNNNNNNNN 1080
   NNNNNNNNNN NNNNNNNNNN NNNNNNNNNN NNNNNNNNNN NNNNNNNNNN NNNNNNNNNN 1140
   NNNNNNNNNN NNNNNNNNNN NNNNNNNNNN NNNNATTTCG GCACAGGTNA AACACTTGAA 1200
   TTTATATTGT ATGGTAGTAT ACTTGGTAAG ATAAAATTCC ACAAAAATAG GGATGGTGCA 1260
   GCATATGCAA TTTCCATTCC TATTATAATT GACACAGTAC ATTAACAATC CATGCCAACG 1320
   GTGCTAATAC GATAGGCTGA NNNNNNNNNN NNNNNNNNNN NNNNNNNNNN NNNNNNNNNN 1380
   NNNNNNNNNN NNNNNNNNNN NNNNNNNNNN NNNNNNNNNN NNNNNNNNNN NNNNNNNNNN 1440
   NNNNNNNNNN NNNNNNNNNN NNNNNNNNNN NNNNNNNNNN NNNNNNNNNN NNNNNNNNNN 1500
   NNNNNNNNNN NNNNNNNNNN NNNNNNNNNN NNNNNNNNNN NNNNNNNNNN NNNNNNNNNN 1560
   NNNNNNNNNN NNNNNNNNNN NNNNNNNNNN NNNNNNNNNN NNNNNNNNNN NNNNNNNNNN 1620
   NNNNNNNNNN NNNNNNNNNN NNNNNNNNNN NNNNNNNNNN NNNNNNNNNN NNNNNNNNNN 1680
   NNNNNNNNNN NNNNNNNNNN NNNNNNNNNN NNNNNNNNNN NNNNNNNNNN NNNNNNNNNN 1740
   NNNNNNNNNN NNNNNNNNNN NNNNNNNNNN NNNNNNNNNN NNNNNNNNNN NNNNNNNNNN 1800
   NNNNNNNNNN NNNNNNNNNN NNNNNNNNNN NNNNNNNNNN NNNNNNNNNN NNNNNNNNNN 1860
   NNNNNNNNNN NNNNNNNNNN NNNNNNNNNN NNNNNNNNNN NNNNNNNNNN NNNNNNNNNN 1920
   TAAATCTCAA CGTTCCATTA TTTTAATACT TGCAAAAACA TTACTAAGTA TACCAAAATA 1980
   ATTGACTCTA TTATCTGAAA TGAAGAATAA ACTGATGCTA TCTCAACAAT AACTGTTACT 2040
   TTTATTTTAT AATTTGATAA TGAATATATT TCTGCATTTA TTTACTTCTG TTTTGTAAAT 2100
   TGGGATTTTG TTAATCAAAT TTATTGTACT ATGACTAAAT GAAATTATTT CTTACATCTA 2160
   ATTTGTAGAA ACAGTATAAG TTATATTAAA GTGTTTTCAC ATTTTTTTGA AAGAC 2215
(2) INFORMATION FOR SEQ ID NO:8
   (i) SEQUENCE CHARACTERISTICS:
      (A) LENGTH: 375 amino acids
      (B) TYPE: amino acid
      (C) STRANDEDNESS: single
      (D) TOPOLOGY: linear
   (xi) SEQUENCE DESCRIPTION: SEQ ID NO:8
   Met Gln Lys Leu Gln Leu Cys Val Tyr Ile Tyr Leu Phe Met Leu Ile
   1               5                   10                  15
   Val Ala Gly Pro Val Asp Leu Asn Glu Asn Ser Glu Gln Lys Glu Asn
               20                  25                  30
   Val Glu Lys Glu Gly Leu Cys Asn Ala Cys Thr Trp Arg Gln Asn Thr
           35                  40                  45
   Lys Ser Ser Arg Ile Glu Ala Ile Lys Ile Gln Ile Leu Ser Lys Leu
       50                  55                  60
   Arg Leu Glu Thr Ala Pro Asn Ile Ser Lys Asp Val Ile Arg Gln Leu
   65                  70                  75                  80
   Leu Pro Lys Ala Pro Pro Leu Arg Glu Leu Ile Asp Gln Tyr Asp Val
                   85                  90                  95
   Gln Arg Asp Asp Ser Ser Asp Gly Ser Leu Glu Asp Asp Asp Tyr His
               100                 105                 110
   Ala Thr Thr Glu Thr Ile Ile Thr Met Pro Thr Glu Ser Asp Phe Leu
           115                 120                 125
   Met Gln Val Asp Gly Lys Pro Lys Cys Cys Phe Phe Lys Phe Ser Ser
       130                 135                 140
   Lys Ile Gln Tyr Asn Lys Val Val Lys Ala Gln Leu Trp Ile Tyr Leu
   145                 150                 155                 160
   Arg Pro Val Glu Thr Pro Thr Thr Val Phe Val Gln Ile Leu Arg Leu
                   165                 170                 175
   Ile Lys Pro Met Lys Asp Gly Thr Arg Tyr Thr Gly Ile Arg Ser Leu
               180                 185                 190
   Lys Leu Asp Met Asn Pro Gly Thr Gly Ile Trp Gln Ser Ile Asp Val
           195                 200                 205
   Lys Thr Val Leu Gln Asn Trp Leu Lys Gln Pro Glu Ser Asn Leu Gly
       210                 215                 220
   Ile Glu Ile Lys Ala Leu Asp Glu Asn Gly His Asp Leu Ala Val Thr
   225                 230                 235                 240
   Phe Pro Gly Pro Gly Glu Asp Gly Leu Asn Pro Phe Leu Glu Val Lys
                   245                 250                 255
   Val Thr Asp Thr Pro Lys Arg Ser Arg Arg Asp Phe Gly Leu Asp Cys
               260                 265                 270
   Asp Glu His Ser Thr Glu Ser Arg Cys Cys Arg Tyr Pro Leu Thr Val
           275                 280                 285
   Asp Phe Glu Ala Phe Gly Trp Asp Trp Ile Ile Ala Pro Lys Arg Tyr
       290                 295                 300
   Lys Ala Asn Tyr Cys Ser Gly Glu Cys Glu Phe Val Phe Leu Gln Lys
   305                 310                 315                 320
   Tyr Pro His Thr His Leu Val His Gln Ala Asn Pro Arg Gly Ser Ala
                   325                 330                 335
   Gly Pro Cys Cys Thr Pro Thr Lys Met Ser Pro Ile Asn Met Leu Tyr
               340                 345                 350
   Phe Asn Gly Lys Glu Gln Ile Ile Tyr Gly Lys Ile Pro Ala Met Val
           355                 360                 365
   Val Asp Arg Cys Gly Cys Ser
       370                 375
(2) INFORMATION FOR SEQ ID NO:9
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:9
      GGCCCAACTA TGGATATATT TG 22
(2) INFORMATION FOR SEQ ID NO:10
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:10
      GGTCCTGGGA AGGTTACAGC A 21
(2) INFORMATION FOR SEQ ID NO:11
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (xi) SEQUENCE DESCRIPTION: SEQ ID NO:11
      ATGAACACTC C 11
(2) INFORMATION FOR SEQ ID NO:12
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:12
      CAGCAAAGTC CTTAATGGTA ACAAGC 26
(2) INFORMATION FOR SEQ ID NO:13
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (Xi) SEQUENCE DESCRIPTION: SEQ ID NO:13
      GGGTCACTGA AGAAAACGTC CTG 23
(2) INFORMATION FOR SEQ ID NO:14
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:14
      CCCCATATTA TGGAGATGAA CCG 23
(2) INFORMATION FOR SEQ ID NO:15
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:15
      AGTTCAGGAT GGCAGAATTT CAG 23
(2) INFORMATION FOR SEQ ID NO:16
   (i) SEQUENCE CHARACTERISTICS:
      (A) LENGTH: 23 base pairs
      (B) TYPE: nucleic acids
      (C) STRANDEDNESS: single
      (D) TOPOLOGY: linear
   (xi) SEQUENCE DESCRIPTION: SEQ ID NO:16
      GCAAACTGGG YGGRAGCAAG ACC 23
(2) INFORMATION FOR SEQ ID NO:17
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (Xi) SEQUENCE DESCRIPTION: SEQ ID NO:17
      TTSTTCCTGG GCTTTTATTG AGAC 24
(2) INFORMATION FOR SEQ ID NO:18
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:18
      AAGCCWGAT TTCTGCTTYT TGGAAG 26
(2) INFORMATION FOR SEQ ID NO:19
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:19
      TGCCMAGGCA HCCRCCRTAC TTGAA 25
(2) INFORMATION FOR SEQ ID NO:20
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:20
      GGTCGTCCTA CACCAGAAG 19
(2) INFORMATION FOR SEQ ID NO:21
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (Xi) SEQUENCE DESCRIPTION: SEQ ID NO:21
      GGTTGACATT GTCAAGAACA AG 22
(2) INFORMATION FOR SEQ ID NO:22
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:22
      TCTCMAAAGT CGTCTGTGAC AATC 24
(2) INFORMATION FOR SEQ ID NO:23
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:23
      TGYTCRTTTT CTTTCAGAGT TGC 23
(2) INFORMATION FOR SEQ ID NO:24
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:24
      RCTGGTCCT CTTCACCTCA GAAC 24
(2) INFORMATION FOR SEQ ID NO:25
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:25
      ACATTGTCVG TTCCAAAGCC AAG 23
(2) INFORMATION FOR SEQ ID NO:26
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:26
      AGGTYCGGGT GACDGTGCTK C 21
(2) INFORMATION FOR SEQ ID NO:27
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:27
      TGGRTACATG AGYTCCACCT TGC 23
(2) INFORMATION FOR SEQ ID NO:28
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:28
      AGCTGCARGT ATWCCTACAA YCT 23
(2) INFORMATION FOR SEQ ID NO:29
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:29
      GTYCCRTTGC TCYTCTCRTT GYC 23
(2) INFORMATION FOR SEQ ID NO:30
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:30
      AACTGTATAT TGAGAGCCTA CCATG 25
(2) INFORMATION FOR SEQ ID NO:31
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:31
      CACACCTTAG CGACTAAACC ACCA 24
(2) INFORMATION FOR SEQ ID NO:32
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:32
      CTACCTAACA GAATGATTTT GTAAG 25
(2) INFORMATION FOR SEQ ID NO:33
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:33
      AGTGTTCTTG CCTAGAGAAT CCCAG 25
(2) INFORMATION FOR SEQ ID NO:34
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:34
      ACATTCTCTC ACCAATATGA CATAC 25
(2) INFORMATION FOR SEQ ID NO:35
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:35
      TAAGTCACCA TTACATCCTT AGAAC 25
(2) INFORMATION FOR SEQ ID NO:36
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:36
      GCTGTAAGAA TCTTCATTAA GCACT 25
(2) INFORMATION FOR SEQ ID NO:37
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:37
      CCTGATACAT GCTAAGGTTA AAAAC 25
(2) INFORMATION FOR SEQ ID NO:38
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:38
      AGGCATACAT CTGGAGAGAA ACATG 25
(2) INFORMATION FOR SEQ ID NO:39
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:39
      CAGAGGAGCC TAGCAGGCTA CCGTC 25
(2) INFORMATION FOR SEQ ID NO:40
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:40
      CAGCAGGTCT GTTGAAGTGT ATCAG 25
(2) INFORMATION FOR SEQ ID NO:41
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:41
      AGTGGTAGCA TTCACAGGTA GCCAG 25
(2) INFORMATION FOR SEQ ID NO:42
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:42
      CAGTCCATGG CACCATAAAG 20
(2) INFORMATION FOR SEQ ID NO:43
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:43
      TCCGTTAGTA CTGGCTAATT GC 22
(2) INFORMATION FOR SEQ ID NO:44
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:44
      CTGAATTGGC TCCAAAGGCC 20
(2) INFORMATION FOR SEQ ID NO:45
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:45
      AAACAGAAGT CCAGGGCTGC 20
(2) INFORMATION FOR SEQ ID NO:46
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:46
      TCAGTCTCCA GGAGAGAAAA C 21
(2) INFORMATION FOR SEQ ID NO:47
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:47
      CTCTGCCCTG GGGATGATTG 20
(2) INFORMATION FOR SEQ ID NO:48
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:48
      AATGTATGTT TATATTTACC TGTTCATG 28
(2) INFORMATION FOR SEQ ID NO:49
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:49
      ACAGTGTTTG TGCAAATCCT GAGAC 25
(2) INFORMATION FOR SEQ ID NO:50
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:50
      CAATGCCTAA GTTGGATTCA GGTTG 25
(2) INFORMATION FOR SEQ ID NO:51
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:51
      CTTGCTGTAA CCTTCCCAGG ACCAG 25
(2) INFORMATION FOR SEQ ID NO:52
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:52
      TCCCATCCAA AGGCTTCAAA ATC 23
(2) INFORMATION FOR SEQ ID NO:53
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:53
      ATACTCWAGG CCTAYAGCCT GTGGT
(2) INFORMATION FOR SEQ ID NO:54
   (i) SEQUENCE CHARACTERISTICS:
      (A) LENGTH: 5790 base pairs
      (B) TYPE: nucleic acid
      (C) STRANDEDNESS: single
      (D) TOPOLOGY: linear
   (xi) SEQUENCE DESCRIPTION: SEQ ID NO:54
   GCGGCCGCCC GGGCAGGTAT CGAAAGTTTC ACATATAAAG ATGAATAAGA TCTAAGTGTA    60
   TATGTTATTG TTAATAAAGT TTTTAATTTT TCGAATGTCA CATACAGCCT TTATTATTCA   120
   TAGATTTATT CCTTTTAAGA AGTAGTCAAA TGAATCAGCT CACCCTTGAC TGTAACAAAA   180
   TACTGTTTGG TGACTTGTGA CAGACAGGGT TTTAACCTCT GACAGCGAGA TTCATTGTGG   240
   AGCAAGAGCC AATCACAGAT CCCGACGACA CTTGTCTCAT CAAAGTTGGA ATATAAAAAG   300
   CCACTTGGAA TACAGTATAA AAGATTCACT GGTGTGGCAA GTTGTCTCTA GACTGGGCAG   360
   GCATTAACGT TTGGCTTGGC GTTACTCAAA AGCAAAAGAA AAGTAAAAGG AAGAAGTAAG   420
   AACAAGGGAA AAGATTGTAT TGATTTTAAA ACC ATG CAA AAA CTG CAA ATC TCT     474
                                        Met Gln Lys Leu Gln Ile Ser
                                        1               5
   GTT TAT ATT TAC CTA TTT ATG CTG ATT GTT GCT GGC CCA GTG GAT CTG     522
   Val Tyr Ile Tyr Leu Phe Met Leu Ile Val Ala Gly Pro Val Asp Leu
       10                  15                  20
   AAT GAG AAC AGC GAG CAG AAG GAA AAT GTG GAA AAA GAG GGG CTG TGT     570
   Asn Glu Asn Ser Glu Gln Lys Glu Asn Val Glu Lys Glu Gly Leu Cys
       25                  30                  35
   AAT GCA TGT TTG TGG AGG GAA AAC ACT ACA TCC TCA AGA CTA GAA GCC     618
   Asn Ala Cys Leu Trp Arg Glu Asn Thr Thr Ser Ser Arg Leu Glu Ala
   40                  45                  50                  55
   ATA AAA ATC CAA ATC CTC AGT AAA CTT CGC CTG GAA ACA GCT CCT AAC     666
   Ile Lys Ile Gln Ile Leu Ser Lys Leu Arg Leu Glu Thr Ala Pro Asn
                   60                  65                  70
   ATC AGC AAA GAT GCT ATC AGA CAA CTT TTG CCC AAG GCT CCT CCA CTC     714
   Ile Ser Lys Asp Ala Ile Arg Gln Leu Leu Pro Lys Ala Pro Pro Leu
               75                  80                  85
   CTG GAA CTG ATT GAT CAG TTC GAT GTC CAG AGA GAT GCC AGC AGT GAC     762
   Leu Glu Leu Ile Asp Gln Phe Asp Val Gln Arg Asp Ala Ser Ser Asp
           90                  95                  100
   GGC TCC TTG GAA GAC GAT GAC TAC CAC GCC AGG ACG GAA ACG GTC ATT     810
   Gly Ser Leu Glu Asp Asp Asp Tyr His Ala Arg Thr Glu Thr Val Ile
       105                 110                 115
   ACC ATG CCC ACG GAG T GTGAGTAGTC CTGCTGGTGC AAAGCAACGA CTCTGCTGAC   866
   Thr Met Pro Thr Glu
   120
   TGCTGTTCTA GTGTTCATGA AAAACCGATC TATTTTCAGG CTCTTTTAAC AAGCTGCTGG   926
   CTTGTATGTA AGGAGGAGGG GAAAGAGCTT TTTTCAAGAT TTCATGAGAA ATAGACCAAT   986
   GAGACTGAAA GCTGCTACTT TATTTGTTTC CTTAGAGAGC TAAAAAGCTA AAAATCAAAA  1046
   ATGAAATGCT TGCATAGCAT TCATGTTATA TAGTTTAGTA TGACAACTAT AACATGTTTA  1106
   TGTTTTCACA GCTTAATGCT ACCAAGGTAA AGGATTGGGA AACAGTATCA GCAATGTGAA  1166
   AAATTTACAT CAAATTTCCT AATTGCATTT GGTTGCCTGA AATATGCATT TATAATAACA  1226
   GGTTTTTTTT TTTTCATTAA TAAAAGAGAA AGGAAGAAAT CTGTAGAGGT TGAAGCCTAT  1286
   CTGGGCATTT GCTGAACACT TAGAATGACT TCTGTTATTC AAAACTATTT CTCATAGGGT    1346
   TTTTATGGTC TTCACAGAGT ATCTAATTTT GAAAGCTATT AGAGTGGAAA GGATAAAAGA    1406
   ATATTCTTAA TAAACTTAAT GTATTAGTAA GAGCAATAAG GAAGTAAACA CAGCATAGTG    1466
   AAAAATCATG AGCTAATCAG CAGAAAATTC TAAGAAATAA ACATTTTAAT TACAAAGTTC    1526
   CACTTATACC CTGACCATGG TACTATTGTT GAGAGTACCT TGTCTGCACA TATCTAGGAG    1586
   GCACATGCTT AATAACCTTC TAAAATATTA TTGTATTCCT CATAGGAGGG AGAACTATTA    1646
   CCTATATGTA GTACCTATGT TGTTTCTGAA AGATAATATG TTTCATGTAT TTCTGTTGCA    1706
   GTCACTTCAA ACCTATACTC AAGGAAAGGG AGACAGGCAT CTCAACAGAG AAGGCATGAC    1766
   CAGAAAGAGT TTTGTGCCAT GTGTCTGCGA TCTTGCTTTA TACAGGGCTC TACCCACTTT    1826
   AAACTGGACT CAAAACAGTT TCAAAATACT GCTTTTTCTT ATTAAGTAAC TAGTTTATAA    1886
   GGCAACAAAT AAATTTCCTT TAAGACTGTG CTATCAGATA ATCCTGGAAT AGATTTGCCT    1946
   TACTTATAAA CAATCTTGAG AAAACAAAAA GGCAAGAAAT TGCTAAGTGC TTCTGCTTAC    2006
   AATGACAGCC TGGCCCTAAA GACAATGTTT TCTAAGTTTT GAAACAGCTT GAATACAACA    2066
   TCTAAGTTTT GGTGCTAATT ACCTGCTAGT TTTTTTATTT TTTTCCTTTA AAAGGCTGTC    2126
   CCAGCGTCCT AACATAACAG ATGCACTATA TTTTCTGCTA ATTCCCGAGG CTCAGTTAGT    2186
   TGCTCACTGT GTCTTGTCCC CAGGTAATTC AGGCCTGGGG GAAGGGTTCC TTCCTCCAGA    2246
   CTGATTGGTA CAGCTGCTCA GTAAGTGTAA CTACTCAGAT TCCCAAAGAA TTCTAAGTGG    2306
   ATGTTCTTCC ACAGTGTCTC TTGTTCTCTC TAATCATCAT CATTTTAAAA TTTCATCCAC    2366
   TTTTCATTCC TTAATAGAAT TTTCCTTAGT CCACAGTTCT CTGGAAAGGA AGTAGGCTTC    2426
   TCATAACAGC TGAAAAAACA TATACCTAAA AGATTCTGAA AAGCTGTAAT AACTGTTATA    2486
   CTTGATATTT TGCTGTTATG AATGAAATGC TACATATTTT TCCATTTTAA AAGACTAAAT    2546
   ATGCACACAT TATTCCAATT AAAAAATGTT CATAGATTGA TATGGAGGTG TTCGTTCATT    2606
   TTTCATAAAA ATGATCTTAG TAACTTTTTT TCTTATTCAT TTATAG CT GAT CTT CTA    2663
                                                    Ser Asp Leu Leu
                                                    125
   ACG CAA GTG GAA GGA AAA CCC AAA TGT TGC TTC TTT AAA TTT AGC TCT      2711
   Thr Gln Val Glu Gly Lys Pro Lys Cys Cys Phe Phe Lys Phe Ser Ser
       130                 135                 140
   AAG ATA CAA TAC AAT AAA CTA GTA AAG GCC CAA CTG TGG ATA TAT CTG      2759
   Lys Ile Gln Tyr Asn Lys Leu Val Lys Ala Gln Leu Trp Ile Tyr Leu
   145                 150                 155                 160
   AGG CCT GTC AAG ACT CCT GCG ACA GTG TTT GTG CAA ATC CTG AGA CTC      2807
   Arg Pro Val Lys Thr Pro Ala Thr Val Phe Val Gln Ile Leu Arg Leu
                   165                 170                 175
   ATC AAA CCC ATG AAA GAC GGT ACA AGG TAT ACT GGA ATC CGA TCT CTG      2855
   Ile Lys Pro Met Lys Asp Gly Thr Arg Tyr Thr Gly Ile Arg Ser Leu
               180                 185                 190
   AAA CTT GAC ATG AAC CCA GGC ACT GGT ATT TGG CAG AGC ATT GAT GTG      2903
   Lys Leu Asp Met Asn Pro Gly Thr Gly Ile Trp Gln Ser Ile Asp Val
           195                 200                 205
   AAG ACA GTG TTG CAG AAC TGG CTC AAA CAA CCT GAA TCC AAC TTA GGC        2951
   Lys Thr Val Leu Gln Asn Trp Leu Lys Gln Pro Glu Ser Asn Leu Gly
       210                 215                 220
   ATT GAA ATC AAA GCT TTA GAT GAG AAT GGC CAT GAT CTT GCT GTA ACC        2999
   Ile Glu Ile Lys Ala Leu Asp Glu Asn Gly His Asp Leu Ala Val Thr
   225                 230                 235                 240
   TTC CCA GAA CCA GGA GAA GAT GGA CTG GTAAGTGATT ACTGAAAATA              3046
   Phe Pro Glu Pro Gly Glu Asp Gly Leu
                   245
   ACATGCTAAA AACCTTGTTA TGTGTTTATT CATAATGTGA ATGAATAGTA GTGAAAAATA      3106
   ACTACCAGTT TCCTGTGCTT ATAAGCCAGA CAAAGGCACC TTACCCCAGT GGTAGCCCTG      3166
   TACTCAATAA AAGTAGGTGT CCCATTTCAC ATCCTATGAA ACACTCTCTT GATACTTTGA      3226
   CTTTGCATGA GGATTTAAAA GAAAAAAAGT TATACCATGG TCCTTAAGTT TTTAGGGAAT      3286
   TCTTTGGAAT TGAGAATGAA ATATAAAATG CTTTCCGTTG ATGTGCTACA TGATTATATA      3346
   AATAAAAACA TGAAGTCTTC ACAGTGGATT CTAGTACTCA CCCAACAACA CATTTTTTCC      3406
   CCCAGAAGAG TGACCAATTT GTTAAAATTC TTTTGCTTAA TAAGGCAGAA AAATGAACTC      3466
   TACAAGTTAT AATTAAAATA AAATGCTTTT ACTTATAGAA ATTAACTAGA TATATGTTCA      3526
   GGTTTATATA CTATTAAATA TACTATATTT AAGATCTCTC ATGATAAATA TGTTCCTTGT      3586
   TTTATAGACT ATTGATGCAC TGATGTATAT GTGGATTACT TTGTGAATTA CCCCTGGTAA      3646
   AATTAAAAAT TTCAGGCTAG TTAACTTGTA CTACTTAGCT ATTTTCTGAA CTGTCTTACT      3706
   GTTCTTTAAC AGGAGTTAAC TTAGGTAATG TCAACTAATT TAATATAAAG TCAAACAGAA      3766
   AATAATGCCT TATATATTAT AAAAATTAAT AAAAAACCAT TTTAAAATCT AGTATAAGTT      3826
   TAGAGCTACT CACTCTTCTG GCTTATCTAT GCTTGTATTT ACTTCTGTTT TCAAAAAATT      3886
   TTTTAATGTG ACCATACCTT TTATTTCCAG TTATTGATAT AATTTACAAC AAAAGATTAT      3946
   ACTTGCAAGC TTTATAGTTT TTAAATGGTC TTATTTGTAG TGAATATCAT ATCTAAATGA      4006
   TATCTAAATG TAAAGTAAAT CATACCTAAA TGAAAACATA TTCTTTAAGT CATTATAAAA      4066
   TTTTCCAGGT GATCAATTTT TCTTTAAATA TACTACATAA AATGTTATTG ACTCCCAAAA      4126
   TGATGTTATT TTGTATAATC TTAAATACCA ATAATTACCA GGTCTATTTT GGTTTTAGTG      4186
   TAGGATAAAA AAGAATGTGT TCTTTTTTCT AGGTAGCATT TTAATGATCA AAGTTGGTGA      4246
   CGTGACAGAG GTCTTAAGTA TTATTAAACA GATGATTAAT AAGATGTATT CCTCAGACTT      4306
   TTCCATATAA AAGGAAAAAT GTCTCAAATT CATGAAAAGA TTGGTACAGG AGGAGGATTA      4366
   GCAAATTGTA GTTTAAATAT CTGAATGGAA ACACTTTTTA GTGAAAGAAT AAAGGGAATA      4426
   TCATTGTATC TTCTTCTGAG TCTGTGCCTC TCTCTCTTGG AGTTAGTCTT TCCAACCCTA      4486
   TATACTTACC ACTATCTTCA TCCCTCTACC TTCCTTTTTC CCATTACATC TGTGCAGTAC      4546
   TGGGTGGCAA CTATTGTGTT TCGGTGTTAA TATCCAAGTT TCCCTGAATA AGACCAAGTG      4606
   AATGGAGGAT GAATGAGTAT ACCTATCCCT CCAGGGGTCA TCAGACATAT TTAGCCACCA      4666
   TATTTAATCA ATAAGCAGGA AGACATAAGC TAGCCTTGTC CTTCTTCTTT CCTCCCTGCT      4726
   CCTTTCTCTT CTCTTCCCCC TCTCCCTTTA CTGTCATCCA TCAGTATTTT CAGAGCATCT      4786
   ATTATGTGTC AGGCATTCAG ATACTCAAAC GGAGGAAAAC AAGAATAAAC AAGACAAAGA        4846
   TCTGACCACA GGGGAATCCC TATGGCTACT GTAGACTTTT GAGCCATAAA GGAAGAATCA        4906
   AGCCTAGTGT AAATGAAAAT TCCTTAATGC TGTGCCTTTT AAAAAGAAAT GTGACATAAG        4966
   CAAAATGATT AGTTTCTTTC TTTAATAATG AGTCCTTGAG GTAGGAGAGT GTTTTGGGAT        5026
   CTATTATTAA CTCTTCTTTC CTTTCCATAC AG ACT CCT TTT TTA GAA GTC AAG          5079
                                       Thr Pro Phe Leu Glu Val Lys
                                       250                 255
   GTA ACA GAC ACA CCA AAA AGA TCT AGG AGA GAT TTT GGG CTT GAT TGT          5127
   Val Thr Asp Thr Pro Lys Arg Ser Arg Arg Asp Phe Gly Leu Asp Cys
               260                 265                 270
   GAT GAA CAC TCC ACA GAA TCT CGA TGC TGT CGT TAC CCT CTA ACT GTG          5175
   Asp Glu His Ser Thr Glu Ser Arg Cys Cys Arg Tyr Pro Leu Thr Val
           275                 280                 285
   GAT TTT GAA GCT TTT GGA TGG GAT TGG ATT ATT GCA CCT AAA AGA TAT          5223
   Asp Phe Glu Ala Phe Gly Trp Asp Trp Ile Ile Ala Pro Lys Arg Tyr
       290                 295                 300
   AAG GCC AAT TAC TGC TCT GGA GAA TGT GAA TTT GTA TTT TTG CAA AAG          5271
   Lys Ala Asn Tyr Cys Ser Gly Glu Cys Glu Phe Val Phe Leu Gln Lys
   305                 310                 315                 320
   TAT CCT CAT ACC CAT CTT GTG CAC CAA GCA AAC CCC AGA GGT TCA GCC          5319
   Tyr Pro His Thr His Leu Val His Gln Ala Asn Pro Arg Gly Ser Ala
                   325                 330                 335
   GGC CCC TGC TGT ACT CCT ACA AAG ATG TCT CCA ATT AAT ATG CTA TAT          5367
   Gly Pro Cys Cys Thr Pro Thr Lys Met Ser Pro Ile Asn Met Leu Tyr
               340                 345                 350
   TTT AAT GGC GAA GGA CAA ATA ATA TAC GGG AAG ATT CCA GCC ATG GTA          5415
   Phe Asn Gly Glu Gly Gln Ile Ile Tyr Gly Lys Ile Pro Ala Met Val
           355                 360                 365
   GTA GAT CGC TGT GGG TGT TCA TGAGGTCTAT ATTTGGTTCA TAGCTTCCTC             5466
   Val Asp Arg Cys Gly Cys Ser
       370                 375
   AAACATGGAA GGTCTTCCCC TCAACAATTT TGAAACTGTG AAATTATGTA CCACAGGCTA        5526
   TAAGCCTAGA GTATGCTACA GTCACTTAAG CACAAGCTAC AGTATATGAG CTAAAAAGAG        5586
   AGAATATATG CAATGGTTGG CATTTAACCA TCCAAACAAA TCGTATAATA AAAAGTTTTA        5646
   TGATTTCCAG AGTTTTTGAA CTAGGAGATC AAATTCCATT TATGTTGAAA TATATTACAA        5706
   CACATGCAGG TGAATGAAAG CAATTCTCCT TGTCTTCTGG TGAATTAAAG GAGTATGCTT        5766
   TAAAATCTAT TTCTCTACAG TTTC                                               5790

## Description

### Field of The Invention

This invention relates to factors affecting muscle development in mammals, especially livestock. In particular, this invention relates to the cloning of a method for determining myostatin genotypes.

### Description Of Related Art

The TGF-β superfamily consists of a group of multifunctional polypeptides which control a wide range of differentiation processes in many mammalian cell types. GDF-8 is a member of the TGF-β superfamily. All members of this superfamily share a common structure including a short peptide signal for secretion and an N-terminal peptide fragment that is separated from the bioactive carboxy-terminal fragment by proteolytic cleavage at a highly conserved proteolytic cleavage site. The bioactive carboxy-terminal domain is characterized by cysteine residues at highly conserved positions which are involved in intra- and intermolecular disulfide bridges. The functional molecules are covalently linked (via a S-S bond) dimers of the carboxy-terminal domain (Masterson *et al.*, 1996).

Recently, it was reported that mice deficient in the gene coding for GDF-8 were characterized by a generalized muscular hyperplasia (McPherron *et al*., 1997). The GDF-8 deficient mice were produced by gene targeting using homologous recombination in embryonic stem cells, a method referred to as "gene knock-out". The murine generalized muscular hyperplasia appeared to be very similar in its expression to the muscular hyperplasia characterizing "double-muscled" cattle. This observation raised the intriguing possibility that the bovine gene coding for GDF-8 (i.e. the bovine evolutionary homologue of the mouse GDF-8 gene) is involved in the bovine double-muscling phenotype. It also raised the possibility that the human gene coding for GDF-8 (i.e. the human evolutionary homologue of the mouse GDF-8 gene) is involved in regulating muscular development in humans, specifically skeletal muscle genesis. Isolation of the human GDF-8 gene may have therapeutic uses/applications in the treatment of musculodegenerative diseases through upgrading or downgrading the expression of GDF-8.

The occurrence of animals characterized by a distinct generalized muscular hypertrophy, commonly known as "double-muscled" animals, has been reported in several cattle breeds around the world. The first documented description of double-muscled cattle dates back as early as 1807 (Culley, 1807). One of the breeds in which this characteristic has been most thoroughly analyzed is the Belgian Blue Cattle Breed ("Belgian Blue Breed"). This is one of the only breeds where the double-muscled trait has been systematically selected for, and where the double-muscled phenotype is virtually fixed. A comparison of double-muscled and conventional animals within the Belgian Blue Breed, showed an increase in muscle mass by 20% on average, while all other organs were reduced in size (Hanset, 1986 and 1991). The muscular hypertrophy was shown to be an histological hyperplasia affecting primarily superficial muscles, accompanied by a 50% reduction in total lipid content and a reduction in connective tissue fraction as measured by hydroxyproline content (Hanset *et al.,* 1982). Double-muscled animals were shown to have a reduced feed intake with improved feed conversion ratio (Hanset *et a*/*.,* 1987). An important economic benefit of double-muscled animals, in contrast to conventional animals, is the substantial increase in selling price and net income for the farmer (Hanset *et a*/*.,* 1987).

One of the most thorough series of studies on double-muscling is that of Hanset and colleagues in the Belgian Blue Breed. Objective criteria of muscular development, such as dressing-out percentage, lean and fat percentage, plasma and red cell creatine and creatinine concentrations, were measured on nearly 150 randomly selected animals raised in standardized conditions. These studies clearly revealed abnormal, bimodal distributions of the double-muscled phenotype and objectively confirmed the visual classification traditionally performed by breeders on double-muscled and conventional animals. The phenotypic distribution was resolved using a maximum likelihood procedure into two component normal populations with a common variance which revealed mean differences of three to four standard deviations depending on the trait. This suggested the presence of an allele having a major effect on muscular development with a population frequency close to 50% (Hanset and Michaux, 1985b). The most convincing evidence in favour of such an allele, however, came from experimental crosses involving double-muscled Belgian Blue sires and Holstein Friesian dairy cows (the latter animals having very poor muscular development). While F1 offspring showed a phenotypic distribution very similar to their Holstein Friesian dams, backcrossing these F1's to double-muscled sires produced a bimodal BC generation, clearly pointing towards the Mendelian segregation of a recessive *"mh"* (muscular hypertrophy) allele (Hanset and Michaux., 1985a).

The same kind of experimental crosses were subsequently used to perform a whole genome scan using a microsatellite based marker map. To perform the linkage analysis, animals were classified as double-muscled or conventional. Very significant Logarithm of the Odds scores (lodscores) were obtained on chromosome 2 (> 17), and multi point linkage analysis positioned the *mh* locus at the centromeric end of this chromosome, at [2]centimorgan from the nearest microsatellite marker: TGLA44. The corresponding chromosomal region accounted for all the variance of the trait assumed to be fully penetrant in this experiment (Charlier *et al*., 1995).

In humans, genes coding for some forms of muscular abnormalities have been isolated, e.g. muscular dystrophy. The disclosure provides for the gene which regulates the development of skeletal muscle only, as opposed to other types of muscle, e.g. smooth or cardiac muscle. The disclosure may provide an understanding of the role of the GDF-8 gene or its receptor in the regrowth of skeletal muscle in humans which only undergo a hyperplasic response.

### Summary of the Invention

The present inventors have identified and sequenced a gene (cDNA and genomic) encoding a bovine myostatin protein. The nucleic acid coding sequence is identified as SEQ ID NO:1 and the protein sequence is identified as SEQ ID NO:2. The genomic bovine i sequence is identified as SEQ ID NO:54. A mutant gene (SEQ ID NO:3) in which the coding sequence lacks an 11-base pair consecutive sequence (SEQ ID NO:11) of the sequence encoding bovine protein having myostatin activity has been sequenced. It has been shown that cattle of the Belgian Blue breed homozygous for the mutant gene lacking myostatin activity are double-muscled. Other bovine mutations which lead to double-muscling in have also been determined, being identified herein as *nt419(del7-ins10), Q204X, E226X* and *C313Y,* respectively. In one aspect, as defined in the claims, the present invention thus provides a method for determining the presence of muscular hyperplasia in a mammal. The method includes obtaining a sample of material containing DNA from the mammal and ascertaining whether a sequence of the DNA encoding (a) a protein having biological activity of myostatin, is present, and whether a sequence of the DNA encoding (b) an allelic protein lacking the activity of (a), is present. The absence of (a) and the presence of (b) indicates the presence of muscular hyperplasia in the mammal.

Of course, the mutation responsible for the lack of activity can be a naturally occurring mutation, as is the case for the Belgian Blue, Asturiana, Parthenaise or Rubia Gallega breeds, shown here.

The mammal can be a bovine, etc.

There are several methods known for determining whether a particular nucleotide sequence is present in a sample. A common method is the polymerase chain reaction. A preferred aspect of the invention thus includes a step in which ascertaining whether a sequence of the DNA encoding (a) is present, and whether a sequence of the DNA encoding (b) is present includes amplifying the DNA in the presence of primers based on a nucleotide sequence encoding a protein having biological activity of myostatin.

A primer of the present invention, used in PCR for example, is a nucleic acid molecule sufficiently complementary to the sequence on which it is based and of sufficient length to selectively hybridize to the corresponding portion of a nucleic acid molecule intended to be amplified and to prime synthesis thereof under *in vitro* conditions commonly used in PCR. Likewise, a probe of the present invention, is a molecule, for example a nucleic acid molecule of sufficient length and sufficently complementary to the nucleic acid molecule of interest, which selectively binds, under high or low stringency conditions with the nucleic acid sequence of interest for detection thereof in the presence of nucleic acid molecules having differing sequences.

Primers can be based on the sequence identified as SEQ ID NO:7 (human cDNA sequence) or SEQ ID NO:54.

Described is a method for determining the presence of muscular hyperplasia in a mammal which includes obtaining a sample of material containing mRNA from the mammal. Such method includes ascertaining whether a sequence of the mRNA encoding (A) a protein having biological activity of myostatin, is present, and whether a sequence of the mRNA encoding (B) a protein at least partially encoded by a truncated nucleotide sequence corresponding to substantially the sequence of the mRNA and lacking the activity of (A), is present. The absence of (A) and the presence of (B) indicates the presence of muscular hyperplasia in the mammal.

The mRNA encoding (A) and the truncated sequence can correspond to alleles of DNA of the mammal.

Again, if an amplification method such as PCR is used in ascertaining whether a sequence of the mRNA encoding (A) is present, and whether a sequence of the mRNA encoding (B) is present, the method includes amplifying the mRNA in the presence of a pair of primers complementary to a nucleotide sequence encoding a protein having biological activity of myostatin. Each such primer can contain a nucleotide sequence substantially complementary, for example, to the sequence identified as SEQ ID NO:7. The truncated sequence can contain at least 50 consecutive nucleotides substantially corresponding to 50 consecutive nucleotides of SEQ ID NO:7, for example.

In another aspect, the invention is a method for determining the presence of muscular hyperplasia In a mammals described which includes obtaining a tissue sample of containing mRNA of the mammal and ascertaining whether an mRNA encoding a mutant type myostatin protein lacking biological activity of myostatin is present The presence of such an mRNA encoding a mutant type myostatin protein indicates the presence of muscular hyperplasia in the mammal.

In another aspect, the invention provides a method for determining the presence of muscular hyperplasia in a bovine animal. The method includes obtaining a sample of material containing DNA from the animal and ascertaining whether DNA having a nucleotide sequence encoding a protein having biological activity of myostatin is present. The absence of DNA having such a nucleotide sequence indicates the presence of muscular hyperplasia in the animal. Ascertaining whether DNA having a nucleotide sequence encoding a protein having biological activity of myostatin can include amplifying the DNA in the presence of primers based on a nucleotide sequence encoding a protein having biological activity of myostatin.

In particular, the method can be carried out using a sample from an animal in which such a bovine animal not displaying muscular hyperplasia is known to have a nucleotide sequence which is capable of hybridizing with a nucleic acid molecule having the sequence identified as SEQ ID NO:1 under stringent hybridization conditions.

It is possible that ascertaining whether DNA having a nucleotide sequence encoding a protein having biological activity of myostatin is present includes amplifying the DNA in the presence of primers based on a nucleotide sequence encoding the N-terminal and the C-terminal, respectively, of the protein having biological activity of myostatin.

Primers, say first and second primers, can be based on first and second nucleotide sequences encoding spaced apart regions of the protein, wherein the regions flank a mutation known to naturally occur and which when present in both alleles of a such an animal results in muscular hyperplasia.

It can also be that DNA of such an animal not displaying muscular hyperplasia contains a nucleotide sequence which hybridizes under stringent conditions with a nucleotide sequence encoding a protein having a sequence identified as SEQ ID NO:2 and the coding sequence of DNA of a such an animal displaying muscular hyperplasia is known to contain an 11-base pair deletion beginning at base pair no. 821 of the coding sequence, and said first primer is selected to be upstream of the codon encoding glutamic acid no. 275 and the second primer is selected to be downstream of the codon encoding aspartic acid no. 274.

Also, a DNA of such an animal not displaying muscular hyperplasia might contain a nucleotide sequence which hybridizes under stringent conditions with a nucleotide sequence encoding a protein having a sequence identified as SEQ ID NO:2. The coding sequence of DNA of such an animal displaying muscular hyperplasia might be known to contain an 11-base pair deletion beginning at base pair no. 821. A primer can be selected to span the nucleotide sequence including base pair nos. 820 and 821 of the DNA sequence containing the deletion.

The animal can be of the Belgian Blue breed.

In a particular aspect, ascertaining whether DNA having a nucleotide sequence encoding a protein having biological activity of myostatin is present includes amplifying the DNA in the presence of a primer containing at least a portion of a mutation known to naturally occur and which when present in both alleles of a said animal results in muscular hyperplasia.

In another aspect, a method for determining the presence of muscular hyperplasia in a bovine animal is described which includes obtaining a sample of the animal containing mRNA and ascertaining whether an mRNA encoding a protein having biological activity of myostatin is present in the sample. The absence of said mRNA indicates the presence of muscular hyperplasia in the animal.

A sample containing mRNA can be muscle tissue, particularly, skeletal muscle tissue.

In a particular aspect, the invention is a method for determining the presence of double muscling in a bovine animal, involving obtaining a sample of material containing DNA from the animal and ascertaining whether the DNA contains the nucleotide sequence identified as SEQ ID NO:11 in which the absence of the sequence indicates double muscling in the animal.

In a particular aspect, the animal is of the Belgian Blue breed.

In another aspect, the invention is a method for determining the myostatin genotype of a mammal, as may be desirable to know for breeding purposes. The method includes obtaining a sample of material containing nucleic acid of the mammal, wherein the nucleic acid is uncontaminated by heterologous nucleic acid; ascertaining whether the sample contains a (i) nucleic acid molecule encoding a protein having biological activity of myostatin; and ascertaining whether the sample contains an (ii) allelic nucleic acid molecule encoding a protein lacking biological activity of myostatin. The mammal can be bovine.

The invention includes a diagnostic kit, for determining the presence of muscular hyperplasia in a mammal from which a sample containing DNA of the mammal has been obtained. The kit includes first and second primers for amplifying the DNA, the primers being complementary to nucleotide sequences of the DNA upstream and down stream, respectively, of a mutation in the portion of the DNA encoding myostatin which results in muscular hyperplasia of the mammal, wherein at least one of the nucleotide sequences is selected to be from a noncoding region of the myostatin gene. The kit can also includes a third primer complementary to a naturally occurring mutation of a coding portion of the myostatin gene.

A particular diagnostic kit, for determining the genotype of a sample of mammalian genetic material, particularly bovine material includes a pair of primers for amplifying a portion of the genetic material corresponding to a nucleotide sequence which encodes at least a portion of a myostatin protein, wherein a first of the primers includes a nucleotide sequence sufficiently complementary to a mutation of SEQ ID NO:1 to prime amplification of a nucleic acid molecule containing the mutation, the mutation being selected from the group of mutations resulting from: (a) deletion of 11 nucleotides beginning at nucleotide 821 of the coding portion of SEQ ID NO:1; (b) deletion of 7 nucleotides beginning at nucleotide 419 of the coding sequence and insertion of the sequence AAGCATACAA in place thereof; (c) deletion of nucleotide 610 of the coding sequence and insertion of T in place thereof; (d) deletion of nucleotide 676 of the coding sequence and insertion of T in place thereof; and (e) and combinations thereof. The second of the pair of primers is preferably located entirely upstream or entirely downstream of the selected mutation or mutations. In one kit, a first said primer spans mutation (a) and further comprising a third primer which is sufficiently complementary to the nucleotide sequence identified as SEQ ID NO:11 to prime amplification of a nucleic acid molecule containing SEQ ID NO:11. In another (or the same kit), a first said primer is sufficiently complementary to the inserted sequence of mutation (b) to prime amplification of a nucleic acid molecule containing mutation (b) and further comprising a third primer which is sufficiently complementary to the sequence corresponding to the 7 nucleotide deletion of mutation (b) to prime amplification of a nucleic acid molecule containing the 7 nucleotide deletion of mutation (b). In another (or the same kit), a first said primer spans mutation (c) and further comprising a third primer which is sufficiently complementary to the sequence spanning the corresponding region lacking mutation (c) to prime amplification of a nucleic acid molecule lacking mutation (c). In another (or the same kit), a first said primer spans mutation (d) and further comprising a third primer which is sufficiently complementary to the sequence spanning the corresponding region lacking mutation (d) to prime amplification of a nucleic acid molecule lacking mutation (d). In another (or the same kit), a first said primer spans mutation (e) and further comprising a third primer which is sufficiently complementary to the sequence spanning the corresponding region lacking mutation (e) to prime amplification of a nucleic acid molecule lacking mutation (e).

Described is a purified protein having biological activity of myostatin, and having an amino acid sequence identified as SEQ ID NO:2, or a conservatively substituted variant thereof. Described is a purified bovine protein having biological activity of myostatin or a purified human protein (SEQ ID NO:8) having biological activity of myostatin.

Described is an isolated nucleic acid molecule encoding a foregoing protein. Described is an isolated nucleic acid molecule comprising a DNA molecule having the nucleotide sequence identified as SEQ ID NO:1 or SEQ ID NO:3 or SEQ ID NO:7 or which varies from the sequence due to the degeneracy of the genetic code, or a nucleic acid strand capable of hybridizing with at least one said nucleic acid molecule under stringent hybridization conditions.

Disclosed is a probe containing a nucleic acid molecule sufficiently complementary with a sequence identified as SEQ ID NO:1, or its complement, so as to bind thereto under stringent conditions. The probe can be a sequence which is between about 8 and about 1195 nucleotides in length.

Disclosed is a primer composition useful for detection of the presence of DNA encoding myostatin in cattle. The composition can include a nucleic acid primer substantially complementary to a nucleic acid sequence encoding a bovine myostatin. The nucleic acid sequence can be that identified as SEQ ID NO:1.

The invention includes a method for identifying a nucleotide sequence of a mutant gene encoding a myostatin protein of a mammal displaying muscular hyperplasia. The method includes obtaining a sample of material containing DNA from the mammal and probing the sample using a nucleic acid probe based on a nucleotide sequence of a known gene encoding myostatin in order to identify nucleotide sequence of the mutant gene. In a particular approach, the probe is based on a nucleotide sequence identified as SEQ ID NO:1, SEQ ID NO:5 or SEQ ID NO:7. Preferably, the probe is at least 8 nucleic acids in length. The step of probing the sample can include exposing the DNA to the probe under hybridizing conditions and further comprising isolating hybridized nucleic acid molecules. The method can further include the step of sequencing isolated DNA. The method can include the step of isolating and sequencing a cDNA or mRNA encoding the complete mutant myostatin protein. The method can include a step of isolating and sequencing a functional wild type myostatin from the mammal not displaying muscular hyperplasia.

The method can include comparing the complete coding sequence of the complete mutant myostatin protein with, if the coding sequence for a functional wild type myostatin from such a mammal is previously known, (1) the known sequence, or if the coding sequence for a functional wild type myostatin from such a mammal is previously unknown, (2) the sequence determined according to claim 63 or claim 66, to determine the location of any mutation in the mutant gene.

The invention includes a primer composition useful for the detection of a nucleotide sequence encoding a myostatin containing a first nucleic acid molecule based on a nucleotide sequence located upstream of a mutation determined according to a method of the invention and a second nucleic acid molecule based on a nucleotide sequence located downstream of the mutation.

A probe of the invention can include a nucleic acid molecule based on a nucleotide sequence spanning a mutation determined according to the invention.

Described is a method for determining whether a sample of mammalian genetic material is capable of a conferring a phenotype characterized by muscular hyperplasia, comprising ascertaining whether the genetic material contains a nucleotide sequence encoding a protein having biological activity of myostatin, wherein the absence of said sequence indicates the presence of muscular hyperplasia in the animal.

Further embodiments of the invention are as described in the claims.

### Brief Description Of Drawings

In describing particular aspects of the invention, reference is made to the accompanying drawings, in which:

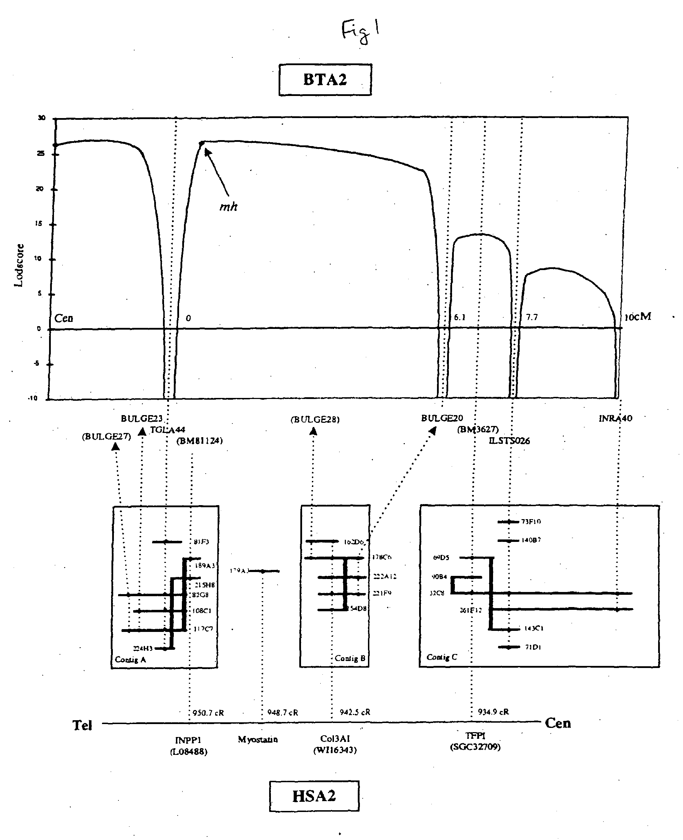

Figure 1 is a schematic summary of genetic, physical and comparative mapping information around the bovine *mh* locus. A multi-point lodscore curve obtained for the *mh* locus with respect to the microsatellite marker map is shown. Markers that were not informative in the pedigree used are shown between brackets; their map position is inferred from published mapping data. Markers and the YACs from which they were isolated are connected by arrows. The RH-map of the relevant section of human HSA2 is shown, with the relative position in cR of the ESTs used. Stippled lines connect microsatellite and Type I markers with their respective positive YACs. YACs showing cross-hybridizing SINE-PCR products are connected by the red boxes.

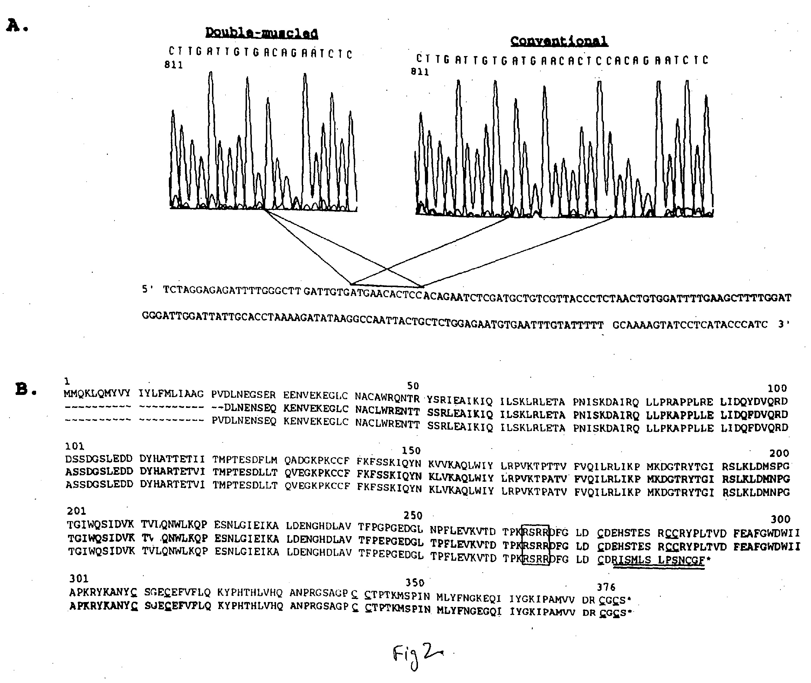

Figure 2(a) shows electropherograms obtained by cycle-sequencing the myostatin cDNA sequence from a double-muscled and a conventional animal, showing the *nt821del(11)* deletion (SEQ ID NO:11) in the double-muscled animal. The primers used to amplify the fragment encompassing the deletion from genomic DNA are spaced apart from the remaining nucleotides.
Figure 2(b) shows the amino-acid sequence of the murine (top row), bovine normal (middle row) and bovine *nt821del(11)* (bottom row) allele. The putative site of proteolytic processing is boxed, while the nine conserved cysteines in the carboxy-terminal region are underlined. The differences between the normal and *nt821del(11)* bovine allele are indicated by the double underlining.

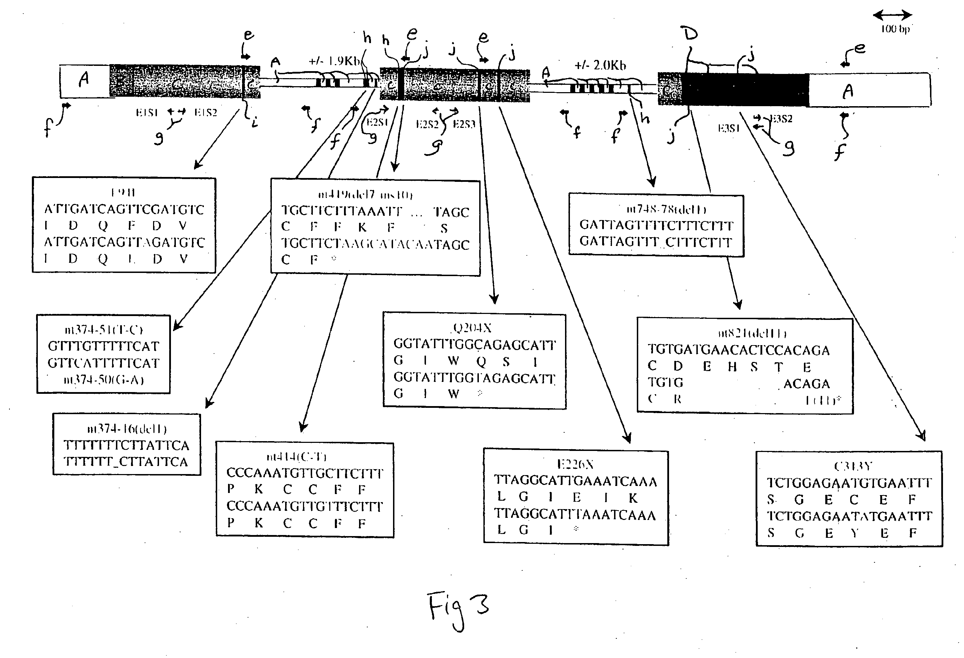

Figure 3 is a schematic representation of the bovine myostatin gene with position and definition of the identified DNA sequence polymorphisms. The "A" (clear) boxes correspond to the untranslated leader and trailer sequences (large diameter), and the intronic sequences (small diameter) respectively. The "B", "C", and "D" boxes correspond to the sequences coding for the leader peptide, N-terminal latency-associated peptide and bioactive carboxyterminal domain of the protein respectively. Small "e", "f" and "g" arrows point towards the positions of the primers used for intron amplification, exon amplification and sequencing and exon sequencing respectively; the corresponding primer sequences are reported in Table 1. The positions of the identified DNA sequence polymorphisms are shown as "h", "i" or "j" lines on the myostatin gene for silent, conservative and disrupting mutations respectively. Each mutation is connected via an arrow with a box reporting the details of the corresponding DNA sequence and eventually encoded peptide sequence. In each box, the variant sequence is compared with the control Holstein-Friesian sequence and differences are highlighted in color.

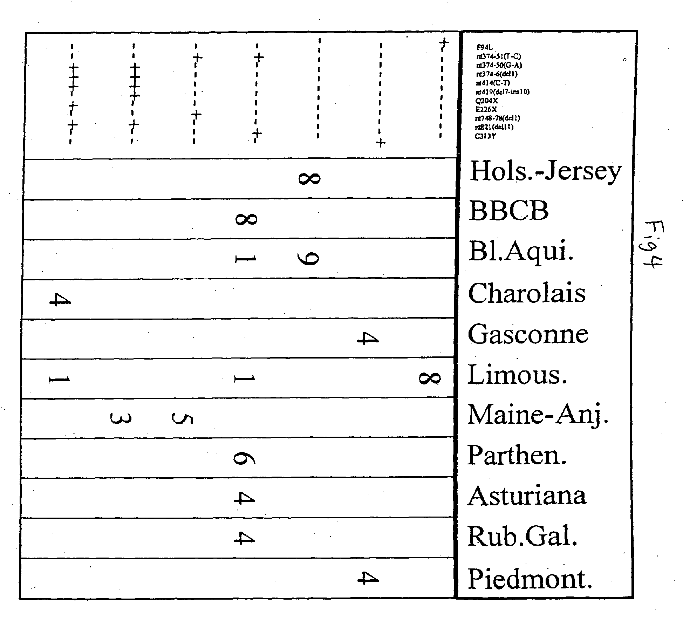

Figure 4 shows the distribution of identified mutations in the various breeds examined. The order of the myostatin mutations correspond to Figure 3. All analyzed animals were double-muscled except for the two Holstein-Friesian and two Jerseys used as controls (column 1).

### Detailed Description Of Preferred Embodiments

The method used for isolating genes which cause specific phenotypes is known as positional candidate cloning. It involves: (i) the chromosomal localization of the gene which causes the specific phenotype using genetic markers in a linkage analysis; and (ii) the identification of the gene which causes the specific phenotype amongst the "candidate" genes known to be located in the corresponding region. Most of the time these candidate genes are selected from available mapping information in humans and mice.

The tools required to perform the initial localization (step (i) above) are microsatellite marker maps, which are available for livestock species and are found in the public domain (Bishop *et al*., 1994; Barendse *et al.,* 1994; Georges *et al*., 1995; and Kappes, 1997). The tools required for the positional candidate cloning, particularly the YAC libraries, (step (ii) above) are partially available from the public domain. Genomic libraries with large inserts constructed with Yeast Artificial Chromosomes ("YAC") are available in the public domain for most livestock species including cattle. When cross-referencing the human and mice map, it is necessary to identify the positional candidate, which is available at low resolution but needs to be refined in every specific instance to obtain the appropriate level of high resolution. A number of original strategies are described herein to achieve this latter objective. For general principles of positional candidate cloning, see Collins, 1995 and Georges and Andersson, 1996.

In order to allow for cross-referencing between the bovine and human gene map as part of the positional candidate cloning approach, HSA2q31-32 (map of the long arm of human chromosome 2, cytogenetic bands q31-32) and BTA2q12-22 (map of the arm of bovine chromosome 2, cytogenetic bands q12-22) were integrated on the basis of coincidence bovine YAC's as described below.

Using a previously described experimental [(normal x double-muscled) x double-muscled] backcross population comprising 108 backcross individuals, the *mh* locus was recently mapped by linkage analysis to the centromeric tip of bovine chromosome 2 (BTA2), at 3.1 centiMorgan proximal from the last marker on the linkage map: TGLA44 (Charlier *et al*., 1995). It was also known from previous work that pro-α(III) collagen (*Col3Al*) was located in the same chromosomal region as the *mh* locus. *Col3Al* has been mapped to BTA2q12-22 by *in situ* hybridization (Solinas-Toldo *et al*., 1995), while a *Col3Al* RFLP marker was shown to be closely linked to TGLA44 (θ=2%)(Fisher *et al.,* 1996). This identifies the region flanking *Col3Al* on the human map, i.e. HSA2q31-32, as the likely orthologous human chromosome segment. This assumption is compatible with data from Zoo-FISH experiments (Solinas-Toldo *et al*., 1995) as well as mapping data of Type I markers on somatic cell hybrids (O'Brien *et al*., 1993), which establish an evolutionary correspondence between segments of HSA2q and BTA2.

In order to refine the correspondence between the HSA2q31-33 and BTA2q12-22 maps, Comparative Anchored Tagged Sequences or CATS, i.e. primer pairs that would amplify a Sequence Tagged Site or STS from the orthologous gene in different species (Lyons *et al.,* 1996), were developed for a series of genes flanking *Col3A1* on the human map and for which sequence information was available in more than one mammal. In addition to *Col3Al,* working CATS were obtained for α2(V) collagen (*Col5A2*), inositol polyphosphate-1 phosphatase (*INPP1*), tissue factor pathway inhibitor precursor (*TFPI*)*,* titin (*TTN*), n-chimaerin (*CHN),* glutamate decarboxylase 67 (*GAD1*), Cytotoxic T-lymphocyte-associated protein 4 (*CTLA4*) and T-cell membrane glycoprotein CD28 (*CD28*). The corresponding primer sequences are given in Table 1.

**Table 1:**

| CATS | | |
|---|---|---|
| INPP1 | UP: 5' CAGCAAAGTCCTTAATGGTAACAAGC 3' | DN: 5' GGGTCACTGAAGAAAACGTCCTG 3' |
| COL3A1 | UP: 5' CCCCATATTATGGAGATGAACCG 3' | DN: 5' AGTTCAGGATGGCAGAATTTCAG 3' |
| COL5A2 | UP: 5' GCAAACTGGGYGGRAGCAAGACC 3' | DN: 5' TTSTTCCTGGGCTTTTATTGAGAC 3' |
| TFPI | UP: 5' AAGCCWGATTTCTGCTTYTTGGAAG 3' | DN: 5' TGCCMAGGCAHCCRCCRTACTTGAA 3' |
| TTN | UP: 5' GGTCGTCCTACACCAGAAG 3' | DN: 5' GGTTGACATTGTCAAGAACAAG 3' |
| CHN | UP: 5' TCTCMAAAGTCGTCTGTGACAATC 3' | DN: 5' TGYTCRTTTTCTTTCAGAGTTGC 3' |
| GAD1 | UP: 5' RCTGGTCCTCTTCACCTCAGAAC 3' | DN: 5' ACATTGTCVGTTCCAAAGCCAAG 3' |
| CTLA4 | UP: 5' AGGTYCGGGTGACDGTGCTKC 3' | DN: 5' TGGRTACATGAGYTCCACCTTGC 3' |
| CD28 | UP: 5' AGCTGCARGTATWCCTACAAYCT 3' | DN: 5' GTYCCRTTGCTCYTCTCRTTGYC 3' |

| Microsatellite markers | | |
|---|---|---|
| TGLA44 | UP:5' AACTGTATATTGAGAGCCTACCATG 3' | DN: 5' CACACCTTAGCGACTAAACCACCA 3' |
| BULGE27 | UP: 5' CTACCTAACAGAATGATTTTGTAAG 3' | DN: 5' AGTGTTCTTGCCTAGAGAATCCCAG 3' |
| BULGE23 | UP: 5' ACATTCTCTCACCAATATGACATAC 3' | DN: 5' TAAGTCACCATTACATCCTTAGAAC 3 |
| BM81124 | UP: 5' GCTGTAAGAATCTTCATTAAGCACT 3' | DN: 5' CCTGATACATGCTAAGGTTAAAAAC 3" |
| BULGE28 | UP: 5' AGGCATACATCTGGAGAGAAACATG 3' | DN: 5' CAGAGGAGCCTAGCAGGCTACCGTC 3' |
| BULGE20 | UP: 5' CAGCAGGTCTGTTGAAGTGTATCAG 3' | DN: 5' AGTGGTAGCATTCACAGGTAGCCAG 3' |
| BM3627 | UP: 5' CAGTCCATGGCACCATAAAG 3' | DN: 5' TCCGTTAGTACTGGCTAATTGC 3' |
| ILSTS026 | UP: 5' CTGAATTGGCTCCAAAGGCC 3' | DN: 5' AAACAGAAGTCCAGGGCTGC 3' |
| INRA40 | UP: 5' TCAGTCTCCAGGAGAGAAAAC 3' | DN: 5' CTCTGCCCTGGGGATGATTG 3' |

| Bovine Myostatin primers | | |
|---|---|---|
| GDF8.19 | 5' AATGTATGTTTATATTTACCTGTTCATG 3' | |
| GDF8.11 | 5' ACAGTGTTTGTGCAAATCCTGAGAC 3' | |
| GDF8.12 | 5' CAATGCCTAAGTTGGATTCAGGTTG 3' | |
| GDF8.25 | 5' CTTGCTGTAACCTTCCCAGGACCAG 3' | |
| GDF8.15 | 5' TCCCATCCAAAGGCTTCAAAATC 3' | |
| GDF8.21 | 5' ATACTCWAGGCCTAYAGCCTGTGGT 3' | |

| | | |
|---|---|---|
| Reading from left to right and down the table, the sequences given in Table 1 are identified as SEQ ID NO:12 to SEQ ID NO:53, respectively. | | |

These CATS were used to screen a 6-genome equivalent bovine YAC library by PCR using a three-dimensional pooling strategy as described by Libert *et al*., 1993. The same YAC library was also screened with all microsatellite markers available for proximaL BTA2, i.e. TGLA44, BM81124, BM3627, ILSTS026, INRA40 and TGLA431 (Kappes *et al.,* 1997).

Potential overlap between the YACs obtained with this panel of STS's was explored on the basis of common STS content, as well as cross-hybridization between SINE-PCR product from individual YACs. From this analysis, three independent YAC contigs emerged in the region of interest: (i) contig A containing microsatellites TGLA44, BM81124 and Type I marker *INPP1;* (ii) contig B containing *Col3Al and Col5A2;* and (iii) contig C containing microsatellite markers BM3627, ILSTS026 and INRA40, and Type I marker *TFPI.*

None of the available microsatellites mapped to contig B, therefore this cluster of YACs could not be positioned in cattle with respect to the two other contigs. Available mapping information in the human, however, allowed prediction of contig B's position between contigs A and C. To test this hypothesis, two new microsatellite markers were isolated from contig B, BULGE20 and BULGE28. BULGE20 proved to be polymorphic, allowing for genotyping of the experimental backcross population.

In addition, to increase the informativeness of the markers available for contig A, two new microsatellite markers were developed from this contig: BULGE23 and BULGE27. BULGE23 proved to be polymorphic and was used to type the same pedigree material.

All resulting genotypes were used to construct a linkage map using the ILINK program (Lathrop and Lalouel, 1984). The following most likely order and sex-averaged recombination rates between adjacent markers was obtained: [TGLA44-(0%)-BULG23]-(6,1%)-BULG20-(1,6%)-ILSTS026-(2.3%)-INRA40-(7,1 %)-TGLA431. The position of BULGE20 between TGLA44 and ILSTS026 confirmed the anticipated order of the three contigs. Figure 1 summarizes the resulting mapping information.

A multi point linkage analysis was undertaken using LINKMAP, to position the *mh* locus with respect to the new marker map. Linkage analysis was performed under a simple recessive model, assuming full penetrance for *mh*/*mh* individuals and zero penetrance for the two other genotypes. The LOD score curve shown in Figure 1 was obtained, placing the *mh* locus in the TGLA44-BULGE20 interval with an associated maximum LOD score of 26.4. Three backcross individuals were shown to recombine with the BULGE20 and distal markers, but not with TGLA44 and BULGE23, therefore placing the *mh* locus proximal from this marker. One individual, was shown to recombine with TGLA44 and BULGE23, but not with the more distal markers, therefore positioning the *mh* locus distal from TGLA44 and BULGE23. Given the relative position of these microsatellite markers with respect to *INPP1* and *Col3Al* as deduced from the integration of the human and bovine map, these results indicated that the *mh* gene is likely located in a chromosome segment bounded by *INPP1* and *Co*/*3Al.*

Recently, McPherron *et al.* (1997) demonstrated that mice homozygous for a knock-out deletion of *GDF-8* or *myostatin,* were characterized by a generalized increase in skeletal muscle mass. Using the published 2676bp murine *myostatin* cDNA sequence (GenBank accession number U84005), a Tentative Human Consensus (THC) cluster in the Unigene database was identified which represented three cDNA clones (221299, 300367, 308202) and six EST (Expressed Sequence Tag) sequences (H92027, H92028, N80248, N95327, W07375, W24782). The corresponding THC covered 1296 bp of the human *myostatin* gene, showing an homology of 78.1 % with the murine sequence when averaged over the entire sequence, and 91.1% when considering only the translated parts of the human and murine genes (566bp). This THC therefore very likely corresponds to the human orthologue of the murine *myostatin* gene. Primers (5'-GGCCCAACTATGGATATATTTG-3' (SEQ ID NO:9) and 5'-GGTCCTGGGAAGGTTACAGCA-3' (SEQ ID NO:10)) were thus prepared to amplify a 272 bp fragment from the second exon of human *myostatin* and used to genotype the whole-genome Genebridge-4 radiation hybrid panel (Walter et al., 1994). The *RHMapper* program (Slonim *et* al., unpublished) was used to position the *myostatin* gene with respect to the Whitehead/MIT framework radiation hybrid map, placing it at position 948.7 cR of the HSA2 map with an associated lodscore > 3. Closer examination of the myostatin segregation vector and its confrontation with the vectors from all markers located in that region (Data Release 11.9, May 1997) showed it to be identical to EST SGC38239 placed on the Whitehead/MIT radiation hybrid map (Hudson *et al.,* 1995) at position 946.8 cR of HSA2. This places the human *myostatin* gene on the RH-map in the interval between *Col3Al* (EST WI16343 - 942.5 cR) and *INPP1* (EST L08488 - 950.2 to 951.2 cR)(Figure 1). *Myostatin* therefore appeared as a very strong positional candidate for the *mh* gene.

To test the potential involvement of *myostatin* in the determinism of double-muscling in cattle, primer pairs were designed based on the available mouse and human *myostatin* sequence, with the objective to amplify the entire coding sequence from bovine cDNA using PCR (Polymerase Chain Reaction). Whenever possible, primers were therefore positioned in portions of the *myostatin* sequence showing 100% homology between mouse and human. Two primer pairs were identified that amplified what was predicted to represent 98.4% of the bovine coding sequence plus 74 bp of 3' untranslated sequence, in two overlapping DNA fragments, respectively 660 (primers GDF8.19 - GDF8.12) and 724 bp (primers GDF8.11 - GDF8.21) long. The expected DNA products were successfully amplified from cDNA generated from skeletal muscle of both a normal (homozygous +/+) (SEQ ID NO:1) and a double-muscled (homozygous *mh*/*mh)* (SEQ ID NO:3) animal, and cycle-sequenced on both strands.

The nucleotide sequence corresponding to the normal allele presented 88.9% identity with the mouse myostatin sequence (SEQ ID NO:5) over a 1067 bp overlap, and contained the expected open reading frame encoding a protein (SEQ ID NO:2) showing 92.9% identity in a 354 amino-acid overlap with mouse myostatin (SEQ ID NO:6). As expected for a member of the TGFβ superfamily, the bovine myostatin gene is characterized by a proteolytic processing site thought to mediate cleavage of the bioactive carboxy-terminal domain from the longer N-terminal fragment, and by nine cysteine residues separated by a characteristic spacing and suspected to be involved in intra- and inter-molecular disulfide bridges (McPherron and Lee, 1996).

The nucleotide sequence obtained from the *mh* allele was identical to the normal allele over its entire length, except for an 11 bp deletion involving nucleotides 821 to 831 (counting from the initiation codon). This frame shifting deletion, occurring after the first cysteine residue of the carboxy-terminal domain, drastically disrupts the downstream amino-acid sequence and reveals a premature stop-codon after 13 amino acids, see Figure 2. The amino acid sequence encoded by the mutant nucleic acid sequence is identified as SEQ ID NO:4. This mutation disrupts the bioactive part of the molecule and is therefore very likely to be the cause of the recessive double-muscling phenotype. Following conventional nomenclature, this mutation will be referred to as *nt829(del11).*

To further strengthen the assumption of the causality of this mutation, primer pairs flanking the deletion (Figure 2) were prepared and the corresponding DNA segment from all animals from the experimental backcross population amplified. PCR was performed in the presence of dCTP³² in order to radioactively label the amplification product. Amplification products were separated on denaturing polyacrylamide gels and detected by autoradiography. A 188 bp product would be expected for the normal allele and a 177 bp product for the *nt821(del11)* allele. Correlation between phenotype and genotype was matched for the entire pedigree. All ten BBCB double-muscled sires were found to be homozygous for the *nt821(del11)* mutation, all 41 F1 females were heterozygous, while 53 double-muscled offspring were homozygous for the mutation, the remaining 55 conventional animals were heterozygous.

To examine the distribution of the *nt821(de*/*11)* mutation in different conventional and double-muscled breeds, a cohort of 25 normal individuals was genotyped representing two dairy breeds (Holstein-Friesian, Red-and -White) and a cohort of 52 double-muscled animals representing four breeds (BBCB, Asturiana, Maine-Anjou and Piémontese). The results are summarized in Table 2. All dairy animals were homozygous normal except for one Red-and-White bull shown to be heterozygous. The occurrence of a small fraction of individuals carrying the mutation in dairy cattle is not unexpected as the phenotype is occasionally described in this breed. In BBCB and Asturiana, all double-muscled animals were homozygous for the *nt821(del11)* deletion, pointing towards allelic homogeneity in these two breeds. Double-muscled Maine-Anjou and Piémontese animals were homozygous "normal", i.e. they did not show the *nt821(del11)* deletion but a distinct cysteine to tyrosine substitution *(C313Y)* in double-muscled Piedmontese animals identified by others *(Kambadur et al.,* 1997) was discovered.

**Table 2:**

| Breed | Phenotype | Genotype | | |
|---|---|---|---|---|
| | | +/+ | *+*/*nt821(del11)* | *nt821(del11)*/*nt821(del11)* |
| Belgian Blue | DM | | | 29 |
| Asturiana | DM | | | 10 |
| Piémontese | DM | 8 | | |
| Maine-Anjou | DM | 4 | | |
| Holstein-Friesian | Normal | 13 | | |
| Red-and-White | Normal | 12 | 1 | |

The entire coding sequence was also determined for the *myostatin* gene in double-muscled individuals from ten European cattle breeds and a series of mutations that disrupt myostatin function were identified.

The coding sequence of four control Holstein-Friesian and Jersey individuals was identical to the previously described wild-type allele (Grobet *et al*., 1997), further indicating that it was the genuine myostatin coding sequence being amplified, and not a non-functional pseudogene.

Amongst the 32 double-muscled animals, seven DNA sequence variants within the coding region were found, as summarized in Figure 3.

In addition to the *nt821(del11)* mutation in the third exon, described above, four new mutations that would be expected to disrupt the myostatin function were found. An insertion/deletion at position 419 counting from the initiation codon, replacing 7 base pairs with an apparently unrelated stretch of 10 base pairs, reveals a premature stop codon in the N-terminal latency-associated peptide at amino-acid position 140. This mutation is referred to as *nt419(del7-ins10).* Two base pair substitutions in the second exon, a C→T transition at nucleotide position 610 and a G→T transversion at nucleotide position 676, each yield a premature stop codon in the same N-terminal latency-associated peptide at amino-acid positions 204 and 226 respectively. These mutations are called *Q204X* and *E226X* respectively. Finally, a G→A transition at nucleotide position 938 results in the substitution of a cysteine by a tyrosine. This mutation is referred to as *C313Y.* This cysteine is the fifth of nine highly conserved cysteine residues typical of the members of the TGF-β superfamily and shared in particular by TGF-β1, - β2 and -β3, and inhibin-βA and -βB (McPherron & Lee, 1996). It is thought to be involved in an intramolecular disulfide bridge stabilizing the three-dimensional conformation of the bioactive carboxyterminal peptide. Its substitution is therefore likely to affect the structure and function of the protein. This *C313Y* has recently also been described by Kambadur *et al.* (1997).

A conservative phenylalanine to leucine substitution was also found at amino-acid position 94 in the first exon, due to a C→A transversion at nucleotide position 282 of the *myostatin* gene. Given the conservative nature of the amino-acid substitution, its location in the less conserved N-terminal latency-associated peptide, and as this mutation was observed at the homozygous condition in animals that were not showing any sign of exceptional muscular development, this mutation probably does not interfere drastically with the myostatic function of the encoded protein, if at all. This mutation is referred to as *F94L.* The murine protein is characterized by a tyrosine at the corresponding amino-acid position.

Also identified was a silent C→T transition at the third position of the 138th cytosine codon in the second exon, referred to as *nt414(C-T).*

In addition to these DNA sequence polymorphisms detected in the coding region of the *myostatin* gene, also found were four DNA sequence variants in intronic sequences which are probably neutral polymorphisms and which have been assigned the following symbols: *nt374-51(T-C), nt374-50(G-A), nt374-16(del1)* in intron 1, and *nt748-78(del1)* in intron 2 (Figure 3).

Figure 4 shows the observed distribution of mutations in the analysed sample sorted by breed. For the majority of the studied breeds, the analyzed double-muscled animals were homozygous for one of the five described mutations expected to disrupt the myostatin function or compound heterozygotes for two of these mutations. This is compatible with the hypothesis that the double-muscled condition has a recessive mode of inheritance in all these breeds.

Only in Limousin and Blonde d'Aquitaine was there no clear evidence for the role of myostatin loss-of-function mutations in the determinism of the observed muscular hypertrophy. Most Limousin animals were homozygous for the conservative F94L substitution which is unlikely to cause the muscular hypertrophy characterizing these animals, as discussed above. One Limousin animal proved to be heterozygous for this mutation, the other allele being the "wild-type" one. All Blonde d'Aquitaine animals were homozygous 'wild-type". These data indicate either that the myostatin gene is possibly not involved in the double-muscled condition characterizing these two breeds, or that there are additional myostatin mutations outside of the coding region. The double-muscling condition is often considered to be less pronounced in Limousin animals compared to other breeds.

The data indicate that some mutations, such as the *nt821del(11)* and *C313Y,* are shared by several breeds which points towards gene migration between the corresponding populations, while others seems to be confined to specific breeds. Moreover, while some breeds (the Belgian Blue breed in particular) seem to be essentially genetically homogeneous others show clear evidence for allelic heterogeneity (e.g. Maine-Anjou).

The observation of allelic heterogeneity contradicts with the classical view that a single mh mutation spread through the European continent in the beginning of the 19th century with the dissemination of the Shorthorn breed from the British Isles (Ménissier, 1982). Two of the mutations at least are shared by more than one breed, indicating some degree of gene migration but definitely not from a single origin.

In mice, and in addition to the *in vitro* generated myostatin knock-out mice (McPherron & Lee, 1997), the *compact* mutation could be due to a naturally occurring mutation at the *myostatin* gene. The *compact* locus has been mapped to the *D1Mit375-D1Mit21* interval on mouse chromosome 1 known to be orthologous to *HSA2q31-32* and *BTA2q12-22* (Varga *et al.,* 1997).

From an applied point of view, the characterisation of a panel of mutations in the *myostatin* gene associated with double-muscling contributes to the establishment of a diagnostic screening system allowing for marker assisted selection for or against this condition in cattle.

### Example 1

### Genetic and physical mapping

Integration of the HSA2q31-32 and BTA2q12-22 maps was done by using coincident YAC's and the *mh* locus was positioned in the interval flanked by Col3Al and INPP1 as follows. Genetic mapping was performed using a previously described (Holstein-Friesian x Belgian Blue) x Belgian Blue experimental backcross population counting 108 informative individuals (Charlier *et al*., 1995). Microsatellite genotyping was performed according to standard procedures (Georges *et al.,* 1995), using the primer sequences reported in Table 1. Linkage analyses were performed with the MLINK, ILINK and LINKMAP programs of the LINKAGE (version 5.1) and FASTLINK (2.3P version, June 1995) packages (Lathrop & Lalouel, 1984; Cottingham *et a*/., 1993). The YAC library was screened by-PCR using a three dimensional pooling scheme as described in Libert *et al*., 1993. The primer pairs corresponding to the CATS used to screen the library are reported in Table 1. Cross-hybridisation between SINE-PCR products of individual YACs was performed according to Hunter *et al.* (1996), using primers reported in Lenstra *et al*. (1993). Microsatellites were isolated from YACs according to Cornelis *et al.* (1992).

### Example 2

### Mapping of the human myostatin gene on the Genebridge-4-panel

DNA from the Genebridge-4 panel (Walter *et al.,* 1994) was purchased from Research Genetics (Huntsville, Alabama), and genotyped by PCR using standard procedures and the following human *myostatin* primer pair (5'-GGCCCAACTATGGATATATTTG-3' and 5'-GGTCCTGGGAAGGTTACAGCA-3'). Mapping was performed via the WWW server of the Whitehead Institute/MIT Center for Genome Research using their *RH-mapper* program (Slonim, D.; Stein, L.; Kruglyak, L.; Lander, E., unpublished) to position the markers with respect to the framework map. Segregation vectors of the query markers were compared with the vectors from all markers in the region of interest in the complete Data Release 11.9 (May 1997) to obtain a more precise position. This positions myostatin in the INPP1-Col3Al on the human map with LOD score superior to 3.

### Example 3

### RT-PCR

To clone the bovine myostatin orthologue a strategy based on RT-PCR amplification from skeletal muscle cDNA was chosen. Total RNA was extracted from skeletal muscle *(Triceps brachialis*) according to Chirgwin *et al.* (1979). RT-PCR was performed using the Gene-Amp RNA PCR Kit (Perkin-Elmer) and the primers reported in Table 1. The PCR products were purified using QiaQuick PCR Purification kit (Qiagen) and sequenced using Dye terminator Cycle Sequencing Ready Reaction (Perkin-Elmer) and an ABI373 automatic sequencer, using the primers reported in Table 2.

### Example 4

### Diagnosis of the nt821(del11) deletion

To diagnose the nt821(del11) the following primer sequences were designed flanking the *nt821(del11)* deletion: 5'-TCTAGGAGAGATTTTGGGCTT-3' (SEQ ID NO:53) and 5-GATGGGTATGAGGATACTTTTGC-3' (SEQ ID NO:52). These primers amplify a 188 bp fragments from normal individuals and a 177bp fragment from double-muscled individuals. Heterozygous individuals show the two amplification products. These amplification products can be detected using a variety of methods. In this example the PCR product was labelled by incorporation of dCTP³², separated on a denaturing acrylamide gel and revealed by autoradiography. Other approaches that could be used to distinguish the three different genotypes are known to those skilled in the art and would include separation in agarose gels and visualization with ethidium bromide, direct sequencing, TaqMan assays, hybridization with allele specific oligonucleotides, reverse dot-blot, RFLP analysis and several others. The specificity of the test is linked to the detected mutation and not to the primers used in the detection method. That means that other primers can easily be designed based on said bovine myostatin sequence that would fulfill the same requirements.

### Example 5

### Determination of mutations in other breeds

A total of 32 animals with extreme muscular development were sampled from ten European beef cattle breeds in which double-muscled animals are known to occur at high to moderate frequency: (i) Belgium: Belgian Blue (4), (ii) France: Blonde d'Aquitaine (5), Charolais (2), Gasconne (2), Limousin (5), Maine-Anjou (4), Parthenaise (3), (ii) Spain: Asturiana (2), Rubia Gallega (2), (iv) Italy: Piedmontese (2). The determination of the double-muscled phenotype of the sampled animals was performed visually by experienced observers. Four animals with conventional phenotype sampled from the Holstein-Friesian (2) and Jersey (2) dairy populations were analysed as controls.

In order to facilitate the study of the myostatin coding sequence from genomic DNA, the sequences of the exon-intron boundaries of the bovine gene were determined. In mice, the *myostatin* gene is known to be interrupted by two introns, respectively ≈ 1.5 and 2.4 Kb long (McPherron & Lee, 1997). Two primer pairs were thus designed, respectively, in bovine exons 1 and 2, and exons 2 and 3, that were predicted to flank the two introns, assuming conservation of gene organisation between mouse and cattle (Figure 3 and Table 3). Using these primer sets, two PCR products respectively 2Kb and 3.5Kb long were generated from a YAC clone (179A3) containing the bovine myostatin gene (Grobet *et al.,* 1997). The PCR products were purified using QiaQuick PCR Purification kit (Qiagen) and partially sequenced using Dye terminator Cycle Sequencing Ready Reaction (Perkin-Elmer) and an ABI373 automatic sequencer. Alignment with the bovine cDNA sequence identified the four predicted exon-intron boundaries. The nucleotide sequence corresponding to bovine genomic DNA is identified as SEQ ID NO:54.

**Table 3: Primers used for PCR amplification and cycle sequencing.**

| | | | |
|---|---|---|---|
| Intron1-5' | | Intron1-3' | |
| Intron2-5' | | Intron2-3' | |
| Exon1-5' | | Exon1-3' | |
| Exon2-5' | | Exon2-3' | |
| Exon3-5' | | Exon3-3' | |
| Exon1-Seq1 | | Exon1-Seq2 | |
| Exon2-Seq1 | | Exon2-Seq2 | |
| Exon2-Seq3 | | | |
| Exon3-Seq1 | | Exon3-Seq2 | |

Based on the available exonic and intronic sequences of the bovine myostatin gene, three primer pairs that jointly allow for convenient amplification of the entire coding sequence from genomic DNA were designed. The position of the corresponding primers is shown in Figure 3, and the corresponding sequences are reported in Table 3.

After PCR amplification of the entire coding sequence from genomic DNA in the three described fragments, these were purified using QiaQuick PCR Purification kit (Qiagen) and sequenced using Dye terminator Cycle Sequencing Ready Reaction (Perkin-Elmer) and an ABI373 automatic sequencer, using the primers used for amplification as well as a series of nested primers (Figure 3 and Table 3). Chromat files produced with the ABI373 sequencer were analysed with the *Polyphred* application (D. Nickerson, personal communication), which is part of a series of sequence analysis programs including *Phred* (Ewing, B. & Green, P. (1992), unpublished), *Phrap* (Green, P. (1994), unpublished) and *Consed (Gordon,* D. (1995), unpublished), but any suitable sequencing programme would do, as known to a person skilled in the art.

Monoclonal antibodies (Mab's) specific for *myostatin* may be useful. In the case of the bovine protein having the amino acid sequence identified as SEQ ID NO:2, for example, antibodies can be used for diagnostic purposes such as for determining myostatin protein levels in muscle tissue. To produce these antibodies, purified myostatin is prepared. The myostatin can be produced in bacterial cells as a fusion protein with glutathione-S-transferase using the vector pGEX2 (Pharmacia). This permits purification of the fusion protein by GSH affinity chromatography. In another approach, myostatin is expressed as a fusion protein with the bacterial maltose binding domain. The fusion protein is thus recovered from bacterial extracts by passing the extract over an amylose resin column followed by elution of the fusion protein with maltose. For this fusion construct, the vector pMalC2, commercially available from New England Biolabs, can be used. The preparation of a second fusion protein can be useful in the preliminary screening of Mab's.

The generation of hybridomas expressing monoclonal antibodies recognizing myostatin protein is carried out as follows: BALB/c mice are injected intraperitoneally with protein/adjuvant three times at one-month intervals, followed by a final injection into the tail vein shortly prior to cell fusion. Spleen cells are harvested and fused with NS-1 myeloma cells (American Type Culture Collection, Rockville, MD) using polyethylene glycol 4000 according to standard protocols (Kennett, 1979; Mirski, 1989). The cell fusion process is carried out as described in more detail below.

The fused cells are plated into 96-well plates with peritoneal exudate cells and irradiated spleen cells from BALB/Ccmice as feeder layers and selection with hypoxanthine, aminopterin, and thymidine (HAT medium) is performed.

An ELISA assay is used as an initial screening procedure. 1-10 µg of purified myostatin (cleaved from the fusion protein) in PBS is used to coat individual wells, and 50-100 µl per well of hybridoma supernatants is incubated. Horseradish peroxidase-conjugated antimouse antibodies are used for the colorimetric assay.

Positive hybridomas are cloned by limiting-dilution and grown to large-scale for freezing and antibody production. Various positive hybridomas are selected for usefulness in western blotting and immunohistochemistry, as well as for cross reactivity with myostatin proteins from different species, for example the mouse and human proteins.

Alternatively, active immunization by the generation of an endogenous antibody by direct exposure of the host animal to small amounts of antigen can be carried out. Active immunization involves the injection of minute quantities of antigen (g) which probably will not induce a physiological response and will be degraded rapidly. Antigen will only need to be administered as prime and boost immunizations in much the same manner as techniques used to confer disease resistance (Pell *et al.,* 1997).

It will of course be understood, that a variety of substitutions of amino acids is possible while preserving the structure responsible for myostatin activity of the proteins disclosed herein. Conservative substitutions are described in the patent literature, as for example, in United States Patent No. 5,264,558 or 5,487,983. It is thus expected, for example, that interchange among non-polar aliphatic neutral amino acids, glycine, alanine, proline, valine and isoleucine, would be possible. Likewise, substitutions among the polar aliphatic neutral amino acids, serine, threonine, methionine, asparagine and glutamine could possibly be made. Substitutions among the charged acidic amino acids, aspartic acid and glutamic acid, could probably be made, as could substitutions among the charged basic amino acids, lysine and arginine. Substitutions among the aromatic amino acids, including phenylalanine, histidine, tryptophan and tyrosine would also likely be possible. These sorts of substitutions and interchanges are well known to those skilled in the art. Other substitutions might well be possible. Of course, it would also be expected that the greater the percentage of homology, i.e., sequence similarity, of a variant protein with a naturally occurring protein, the greater the retention of metabolic activity. Of course, as protein variants having the activity of myostatin as described herein are intended to be within the scope of this invention, so are nucleic acids encoding such variants.

A further advantage may be obtained through chimeric forms of the protein, as known in the art. A DNA sequence encoding the entire protein, or a portion of the protein, could thus be linked, for example, with a sequence coding for the C-terminal portion of *E*. *coli* β-galactosidase to produce a fusion protein. An expression system for human respiratory syncytial virus glycoproteins F and G is described in United States Patent No. 5,288,630 issued February 22,1994 for example.

A recombinant expression vector can be a plasmid, as described above. The recombinant expression vector of the invention further can be a virus, or portion thereof, which allows for expression of a nucleic acid introduced into the viral nucleic acid. For example, replication defective retroviruses, adenoviruses and adeno-associated viruses can be used.

Described is a diagnostic kit for identifying cells comprising a molecule which binds to a protein comprising an amino acid sequence shown in SEQ ID NO:2, for example, for incubation with a sample of tumor cells; means for detecting the molecule bound to the protein, unreacted protein or unbound molecule; means for determining the amount of protein in the sample; and means for comparing the amount of protein in the sample with a standard. The molecule can be a monoclonal antibody.

The detectability of the molecule which binds to myostatin can be activated by said blinding (e.g., change in fluorescence spectrum, loss of radioisotopic label). The diagnostic kit can also contain an instruction manual for use of the kit.

Further disclosed is a diagnostic kit for identifying cells comprising a nucleotide probe complementary to the sequence, or an oligonucleotide fragment thereof, shown in SEQ ID NO:1, for example, for hybridization with mRNA from a sample of cells, e.g., muscle cells; means for detecting the nucleotide probe bound to mRNA in the sample with a standard. In a particular aspect, the invention is a probe having a nucleic acid molecule sufficiently complementary with a sequence identified as SEQ ID NO:1, or its complement, so as to bind thereto under stringent conditions. "Stringent hybridization conditions" takes on its common meaning to a person skilled in the art here. Appropriate stringency conditions which promote nucleic acid hybridization, for example, 6x sodium chloride/sodium citrate (SSC) at about 45°C are known to those skilled in the art. The following examples are found in Current Protocols in Molecular Biology, John Wiley & Sons, NY (1989), 6.3.1-6.3.6: For 50 ml of a first suitable hybridization solution, mix together 24 ml formamide, 12 ml 20x SSC, 0.5 ml 2 M Tris-HCl pH 7.6, 0.5 ml 100x Denhardt's solution, 2.5 ml deionized H₂O, 10 ml 50% dextran sulfate, and 0.5 ml 10% SDS. A second suitable hybridization solution can be 1% crystalline BSA (fraction V), 1 mM EDTA, 0.5 M Na₂HPO₄ pH 7.2, 7% SDS. The salt concentration in the wash step can be selected from a low stringency of about 2x SSC at 50°C to a high stringency of about 0.2x SSC at 50°C. Both of these wash solutions may contain 0.1 % SDS. In addition, the temperature in the wash step can be increased from low stringency conditions at room temperature, about 22°C, to high stringency conditions, at about 65°C. The cited reference gives more detail, but appropriate wash stringency depends on degree of homology and length of probe. If homology is 100%, a high temperature (65°C to 75°C) may be used. If homology is low, lower wash temperatures must be used. However, if the probe is very short (<100bp), lower temperatures must be used even with 100% homology. In general, one starts washing at low temperatures (37°C to 40°C), and raises the temperature by 3-5°C intervals until background is low enough not to be a major factor in autoradiography. The diagnostic kit can also contain an instruction manual for use of the kit. The invention provides a diagnostic kit as defined in the claims which can be used to determine the genotype of mammalian genetic material, for example. One kit includes a set of primers used for amplifying the genetic material. A kit can contain a primer including a nucleotide sequence for amplifying a region of the genetic material containing one of the naturally occurring mutations described herein. Such a kit could also include a primer for amplifying the corresponding region of the normal gene that produces functional myostatin. Usually, such a kit would also include another primer upstream or downstream of the region of interest complementary to a coding and/or non-coding portion of the gene. A particular kit includes a primer selected from a non-coding sequence of a myostatin gene. Examples of such primers are provided in Table 3, designated as Exon1-5', Exon1-3', Exon2-5', Exon3-5' and Exon3-3'. These primers are used to amplify the segment containing the mutation of interest The actual genotyping is carried out using primers that target specific mutations described herein and that could function as allele-specific oligonucleotides in conventional hybridization, Taqman assays, OLE assays, etc. Alternatively, primers can be designed to permit genotyping by microsequencing.

One kit of primers thus includes first, second and third primers, (a), (b) and (c), respectively. Primer (a) is based on a region containing a myostatin mutation, for example a region of the myostatin gene spanning the *nt821del(11)* deletion. Primer (b) encodes a region upstream or downstream of the region to be amplified by primer (a) so that genetic material containing the mutation is amplified, by PCR, for example, in the presence of the two primers. Primer (c) is based on the region corresponding to that on which primer (a) is based, but lacking the mutation. Thus, genetic material containing the non-mutated region will be amplified in the presence of primers (b) and (c). Genetic material homozygous for the wild type gene will thus provide amplified products in the presence of primers (b) and (c). Genetic material homozygous for the mutated gene will thus provide amplified products in the presence of primers (a) and (b). Heterozygous genetic material will provide amplified products in both cases.

Described are purified proteins having biological activity of myostatin. The terms "isolated" and "purified" each refer to a protein substantially free of cellular material or culture medium when produced by recombinant DNA techniques, or chemical precursors or other chemicals when chemically synthesized. In certain aspects, the protein having biological activity of myostatin comprises an amino acid sequence identified as SEQ ID NO:2. Furthermore, proteins having biological activity of myostatin that are encoded by nucleic acids which hybridize under stringent conditions, as discussed above, to a nucleic acid comprising a nucleotide sequence identified as SEQ ID NO:1 or SEQ ID NO:7 are encompassed.

Proteins having myostatin activity can be obtained by expression in a suitable host cell using techniques known in the art. Suitable host cells include prokaryotic or eukaryotic organisms or cell lines, for example, yeast, *E. coli,* insect cells and COS 1 cells.

Disclosed are the following items:
1. A method of increasing muscle mass of a mammal having muscle cells in which myostatin is expressed, the method comprising administering to the mammal an effective amount of a nucleic acid molecule substantial complementary to at least a portion of mRNA encoding the myostatin and being of sufficient length to sufficiently reduce expression of the myostatin to increase the muscle mass.
2. The method of item 1 wherein the mammal is bovine.
3. A method of increasing muscle mass of a mammal, the method comprising administering to the mammal an effective amount of a nucleic acid molecule having ribozyme activity and a nucleotide sequence substantially complementary to at least a portion of mRNA encoding myostatin and being of sufficient length to bind selectively thereto to sufficiently reduce expression of the myostatin so as to increase the muscle mass.
4. The method of item 3 wherein the mammal is bovine.
5. A diagnostic kit, for determining the presence of muscular hyperplasia in a mammal from which a sample containing DNA of the mammal has been obtained, the kit comprising:
   first and second primers for amplifying the DNA, the primers being complementary to nucleotide sequences of the DNA upstream and down stream, respectively, of a mutation in the portion of the DNA encoding myostatin which results in muscular hyperplasia of the mammal, wherein at least one of the nucleotide sequences is selected to be from a non-coding region of the myostatin gene.
6. The diagnostic kit of item 5, further comprising a third primer complementary to a naturally occurring mutation of a coding portion of the myostatin gene.
7. A diagnostic kit, for determining the genotype of a sample of mammalian genetic material, the kit comprising:
   a pair of primers for amplifying a portion of the genetic material corresponding to a nucleotide sequence which encodes at least a portion of a myostatin protein, wherein a first of the primers includes a nucleotide sequence sufficiently complementary to a mutation of SEQ ID NO: 1 to prime amplification of a nucleic acid molecule containing the mutation, the mutation being selected from the group of mutations resulting from: (a) deletion of 11 nucleotides beginning at nucleotide 821 of the coding portion of SEQ ID NO:1; (b) deletion of 7 nucleotides beginning at nucleotide 419 of the coding sequence and insertion of the sequence AAGCATACAA in place thereof; (c) deletion of nucleotide 204 of the coding sequence and insertion of T in place thereof; (d) deletion of nucleotide 226 of the coding sequence and insertion of T in place thereof; and (e) deletion of nucleotide 313 of the coding sequence and insertion of A in place thereof; and combinations thereof.
8. The diagnostic kit of item 7 wherein a second of the pair of primers is located entirely upstream or entirely downstream of the selected mutation or mutations.
9. The diagnostic kit of item 8 wherein a first said primer spans mutation (a) and further comprising a third primer which is sufficiently complementary to the nucleotide sequence identified as SEQ ID NO: 11 to prime amplification of a nucleic acid molecule containing SEQ ID NO:11.
10. The diagnostic kit of item 8 wherein a first said primer is sufficiently complementary to the inserted sequence of mutation (b) to prime amplification of a nucleic acid molecule containing mutation (b) and further comprising a third primer which is sufficiently complementary to the sequence corresponding to the 7 nucleotide deletion of mutation (b) to prime amplification of a nucleic acid molecule containing the 7 nucleotide deletion of mutation (b).
11. The diagnostic kit of item 8 wherein a first said primer spans mutation (c) and further comprising a third primer which is sufficiently complementary to the sequence spanning the corresponding region lacking mutation (c) to prime amplification of a nucleic acid molecule lacking mutation (c).
12. The diagnostic kit of item 8 wherein a first said primer spans mutation (d) and further comprising a third primer which is sufficiently complementary to the sequence spanning the corresponding region lacking mutation (d) to prime amplification of a nucleic acid molecule lacking mutation (d).
13. The diagnostic kit of item 8 wherein a first said primer spans mutation (e) and further comprising a third primer which is sufficiently complementary to the sequence spanning the corresponding region lacking mutation (e) to prime amplification of a nucleic acid molecule lacking mutation (e).
14. A method for determining the presence of muscular hyperplasia in a bovine animal, the method comprising:
   obtaining a sample of material containing DNA from a said animal; and ascertaining whether DNA having a nucleotide sequence encoding a protein having biological activity of myostatin is present, wherein the absence of DNA having said nucleotide sequence indicates the presence of muscular hyperplasia in the animal.
15. The method of item 14 wherein ascertaining whether DNA having a nucleotide sequence encoding a protein having biological activity of myostatin includes amplifying the DNA in the presence of primers based on a nucleotide sequence encoding a protein having biological activity of myostatin.
16. The method of item 15 wherein DNA of a said bovine animal not displaying muscular hyperplasia has a nucleotide sequence which is capable of hybridizing with a nucleic acid molecule having the sequence identified as SEQ ID NO: 1 under stringent hybridization conditions.
17. The method of item 14, wherein ascertaining whether DNA having a nucleotide sequence encoding a protein having biological activity of myostatin is present includes amplifying the DNA in the presence of primers based on a nucleotide sequence encoding the N-terminal and the C-terminal, respectively, of the protein having biological activity of myostatin.
18. The method of item 14, wherein ascertaining whether DNA having a nucleotide sequence encoding a protein having biological activity of myostatin is present includes amplifying the DNA in the presence of first and second primers based on first and second nucleotide sequences encoding spaced apart regions of the protein, wherein said regions flank a mutation known to naturally occur and which when present in both alleles of a said animal results in said muscular hyperplasia.
19. The method of item 18 wherein a DNA of said animal not displaying muscular hyperplasia contains a nucleotide sequence which hybridizes under stringent conditions with a nucleotide sequence encoding a protein having a sequence identified as SEQ ID NO: 2 and the coding sequence of DNA of a said animal displaying muscular hyperplasia is known to contain an 11 base pair deletion beginning at base pair no. 821, and said first primer is selected to be upstream of the codon encoding glutamic acid no. 275 and the second primer is selected to be downstream of the codon encoding aspartic acid no. 274.
20. The method of item 14 wherein a DNA of said animal not displaying muscular hyperplasia contains a nucleotide sequence which hybridizes under stringent conditions with a nucleotide sequence encoding a protein having a sequence identified as SEQ ID NO: 2 and the coding sequence of DNA of a said animal displaying muscular hyperplasia is known to contain an 11 base pair deletion beginning at base pair no. 821, and said primer is selected to span the nucleotide sequence including base pair nos. 820 and 821 of the DNA sequence containing said deletion.
21. The method of item 19 wherein the animal is of a breed selected from Belgian Blue, Asturiana, Parthenaise and Rubia Gallega.
22. The method of item 20 wherein the animal is a breed selected from Belgian Blue, Asturiana, Parthenaise and Rubia Gallega.
23. The method of item 14 wherein ascertaining whether DNA having a nucleotide sequence encoding a protein having biological activity of myostatin is present includes amplifying the DNA in the presence of a primer containing at least a portion of a mutation known to naturally occur and which when present in both alleles of a said animal results in said muscular hyperplasia.
24. A method for determining the presence of muscular hyperplasia in a bovine animal, the method comprising:
   obtaining a sample of material containing DNA from a said animal; and
   ascertaining whether DNA having a mutation as defined in item 7 is present; and
   ascertaining whether DNA having a nucleotide sequence encoding a protein having biological activity of myostatin is present, wherein the absence of DNA having said nucleotide sequence and presence of a said mutation indicates the presence of muscular hyperplasia in the animal.
25. A method for determining the presence of muscular hyperplasia in a bovine animal, the method comprising:
   obtaining a sample of the animal containing mRNA; and
   ascertaining whether an mRNA encoding a protein having biological activity of myostatin is present in the sample, wherein the absence of said mRNA indicates the presence of muscular hyperplasia in the animal.
26. The method of item 25 wherein the sample is of muscle tissue or wherein the tissue is skeletal muscle tissue.
27. The method of item 25 wherein ascertaining whether mRNA having a nucleotide sequence encoding a protein having biological activity of myostatin includes amplifying the mRNA in the presence of primers substantially complementary to the nucleotide sequence encoding the protein.
28. The method of item 27 wherein mRNA of a said bovine animal not displaying muscular hyperplasia has a nucleotide sequence which is capable of hybridizing with a nucleic acid molecule having the sequence identified as SEQ ID NO: 1 under stringent hybridization conditions.
29. The method of item 25, wherein ascertaining whether mRNA having a nucleotide sequence encoding a protein having biological activity of myostatin is present includes amplifying the mRNA in the presence of primers substantially complementary to a nucleotide sequence encoding the N-terminal and the C-terminal, respectively, of the protein having biological activity of myostatin.
30. The method of item 25, wherein ascertaining whether mRNA having a nucleotide sequence encoding a protein having biological activity of myostatin is present includes amplifying the mRNA in the presence of first and second primers substantially complementary to first and second nucleotide sequences encoding spaced apart regions of the protein, wherein said regions flank a mutation known to naturally occur and which when present in both alleles of a said animal results in said muscular hyperplasia.
31. The method of item 30 wherein an mRNA of said animal not displaying muscular hyperplasia contains a nucleotide sequence which hybridizes under stringent conditions with a nucleotide sequence encoding a protein having a sequence identified as SEQ ID NO: 2 and the coding sequence of DNA of a said animal displaying muscular hyperplasia is known to contain an 11-base pair deletion beginning at base pair no. 821, and said first primer is selected to be upstream of the codon encoding glutamic acid no. 275 and the second primer is selected to be downstream of the codon encoding aspartic acid no. 274.
32. The method of item 25 wherein ascertaining whether mRNA having a nucleotide sequence encoding a protein having biological activity of myostatin is present includes amplifying the mRNA in the presence of a primer containing a nucleotide sequence complementary to at least a portion of a mutation known to naturally occur in a said animal and which when present in both alieles of a said animal results in said muscular hyperplasia.
33. The method of item 32 wherein an mRNA of said animal not displaying muscular hyperplasia contains a nucleotide sequence which hybridizes under stringent conditions with a nucleotide sequence encoding a protein having a sequence identified as SEQ ID NO: 2 and the coding sequence of DNA of a said animal displaying muscular hyperplasia is known to contain an 11-base pair deletion beginning at base pair no. 821, and said primer is selected to span the deleted portion.
34. The method of item 31 wherein the animal is of a breed selected from Belgian Blue, Asturiana, Parthenaise and Rubia Gallega.
35. A method for determining the presence of muscular hyperplasia in a mammal, the method comprising:
   obtaining a sample of material containing DNA from the mammal; and
   ascertaining whether a sequence of the DNA encoding (a) a protein having biological activity of myostatin, is present, and whether a sequence of the DNA encoding (b) an allelic protein lacking the activity of (a), is present;
   wherein the absence of (a) and the presence of (b) indicates the presence of muscular hyperplasia in the mammal.
36. The method of item 35 wherein (b) contains a naturally occurring mutation responsible for the lack of activity.
37. The method of item 35 wherein the mammal is a human.
38. The method of item 37 wherein ascertaining whether a sequence of the DNA encoding (a) is present, and whether a sequence of the DNA encoding (b) is present includes amplifying the DNA in the presence of primers based on a nucleotide sequence encoding a protein having biological activity of myostatin.
39. The method of item 38 wherein said primers are based on the sequence identified as SEQ ID NO: 7.
40. A method for determining the presence of muscular hyperplasia in a mammal, the method comprising:
   obtaining a sample of material containing mRNA from the mammal; and
   ascertaining whether a sequence of the mRNA encoding (a) a protein having biological activity of myostatin, is present, and whether a sequence of the mRNA encoding (b) a protein at least partially encoded by a truncated nucleotide sequence corresponding to substantially the sequence of the mRNA and lacking the activity of (a), is present;
   wherein the absence of (a) and the presence of (b) indicates the presence of muscular hyperplasia in the mammal.
41. The method of item 40 wherein the mRNA encoding (a) and the truncated sequence correspond to alleles of DNA of the mammal.
42. The method of item 40 wherein the mammal is human.
43. The method of item 42 wherein ascertaining whether a sequence of the mRNA encoding (a) is present, and whether a sequence of the mRNA encoding (b) is present includes amplifying the mRNA in the presence of a pair of primers complementary to a nucleotide sequence encoding a protein having biological activity of myostatin.
44. The method of item 43 wherein each said primer contains a truncated nucleotide sequence substantially complementary to a portion of the sequence identified as SEQ ID NO: 7.
45. The method of item 44 wherein the truncated sequence contains at least 50 consecutive nucleotides substantially corresponding to about 10, or between about 10 and 20, or between about 20 and 30, or between about 30 and 40, or between about 40 and 50 consecutive nucleotides of SEQ ID NO: 7.
46. A method for determining the presence of muscular hyperplasia in a mammal, the method comprising:
   obtaining a tissue sample of containing mRNA of the mammal; and
   ascertaining whether an mRNA encoding a mutant type myostatin protein lacking biological activity of myostatin is present, wherein the presence of a said mRNA encoding a mutant type myostatin protein indicates the presence of muscular hyperplasia in the mammal.
47. The method of item 46 wherein the mutant type myostatin protein lacing biological activity is encoded by a naturally occurring allele of DNA encoding the mRNA.
48. A method for determining the presence of double muscling in a bovine animal, the method comprising:
   obtaining a sample of material containing DNA from the animal; and
   ascertaining whether the DNA contains the nucleotide coding sequence identified as SEQ ID NO:11,
   wherein absence of the sequence indicates double muscling in the animal.
49. The method of item 34 wherein the animal is of a breed selected from Belgian Blue, Asturiana, Parthenaise and Rubia Gallega.
50. A method for determining the myostatin genotype of a mammal, comprising:
   obtaining a sample of material containing nucleic acid of the mammal, wherein the nucleic acid is uncontaminated by heterologous nucleic acid;
   ascertaining whether the sample contains a (i) nucleic acid molecule encoding a protein having biological activity of myostatin; and
   ascertaining whether the sample contains an (ii) allelic nucleic acid molecule encoding a protein lacking biological activity of myostatin.
51. The method of item 50 wherein the mammal is human and (i) comprises a nucleic acid sequence substantially homologous with the sequence identified as SEQ ID NO: 7.
52. A purified protein having biological activity of myostatin, and having an amino acid sequence identified as SEQ ID NO: 2, or a conservatively substituted variant thereof.
53. An isolated nucleic acid molecule encoding a protein of item 52.
54. An isolated nucleic acid molecule comprising a DNA molecule having the nucleotide sequence identified as SEQ ID NO: 1 or which varies from the sequence due to the degeneracy of the genetic code, or a nucleic acid strand capable of hybridizing with at least one said nucleic acid molecule under stringent hybridization conditions.
55. Isolated mRNA transcribed from DNA having a sequence which corresponds to a nucleic acid molecule according to item 54.
56. Isolated DNA having a sequence according to item 54 in a recombinant cloning vector.
57. A microbial cell containing and expressing heterologous DNA which is complementary a nucleic acid molecule of item 54.
58. A transfected cell line which expresses a protein of item 52.
59. A process for producing the protein of item 52 comprising:
   preparing a DNA fragment including a nucleotide sequence which encodes said protein; incorporating the DNA fragment into an expression vector to obtain a recombinant DNA molecule which includes the DNA fragment and is capable of undergoing replication;
   transforming a host cell with said recombinant DNA molecule to produce a transformant which can express said protein;
   culturing the transformant to produce said protein; and
   recovering said protein from resulting cultured mixture.
60. A method of increasing muscle mass in a mammal, comprising administering an effective amount of an antibody to myostatin to said mammal.
61. A method of increasing muscle mass in a mammal, comprising raising an autoantibody to the myostatin the in the mammal.
62. The method of item 61 wherein raising the autoantibody includes administering a protein having myostatin activity to the mammal.
63. A method of increasing muscle mass in a mammal in need thereof, comprising administering to the mammal an effective amount of an antisense nucleic acid or oligonucleotide substantially complementary to at least a portion of the sequence identified as SEQ ID NO:1 or SEQ ID NO: 5, or SEQ ID NO: 7.
64. The method of item 63 wherein the portion is at least 5 nucleotide bases in length.
65. The method of item 64 wherein the mammal is a bovine and the sequence is the sequence identified as SEQ ID NO: 1.
66. A method of increasing muscle mass in a mammal, comprising administering to the mammal an effective amount of an antibody to the myostatin.
67. A probe comprising a nucleic acid molecule sufficiently complementary with a sequence identified as SEQ ID NO: 1, or its complement, so as to bind thereto under stringent conditions.
68. The probe of item 67 wherein the sequence is between about 8 and about 1195 nucleotides in length, or between about 15 and 1195 nucleotides in length, or between about 25 and 1195 nucleotides in length, or between about 35 and 1195 nucleotides in length, or between about 45 and 1195 nucleotides in length, or between about 55 and 1195 nucleotides in length, or between about 65 and 1195 nucleotides in length, or between about 75 and 1195 nucleotides in length, or between about 75 and 1195 nucleotides in length, or between about 85 and 1195 nucleotides in length, or between about 95 and 1195 nucleotides in length, or between about 105 and 1195 nucleotides in length, or between about 115 and 1195 nucleotides in length.
69. A method for identifying a nucleotide sequence of a mutant gene encoding a myostatin protein of a mammal displaying muscular hyperplasia, the method comprising:
   obtaining a sample of material containing DNA from the mammal; and
   probing the sample using a nucleic acid probe based on a nucleotide sequence of a known gene encoding myostatin in order to identify nucleotide sequence of the mutant gene.
70. The method of item 69, wherein the probe is based on a nucleotide sequence of a noncoding region of the gene.
71. The method of item 70 wherein the probe is based on SEQ ID NO: 54.
72. The method of item 71 wherein the probe is at least 8 nucleic acids in length.
73. The method of item 69, wherein the step of probing the sample includes exposing the DNA to the probe under hybridizing conditions and further comprising isolating hybridized nucleic acid molecules.
74. The method of item 73, further comprising the step of sequencing isolated DNA.
75. The method of item 69, wherein the mammal is a bovine mammal and the probe is based on a said nucleotide sequence identified as SEQ ID NO: 1.
76. The method of item 74, further comprising the step of isolating and sequencing a cDNA or mRNA encoding the complete mutant myostatin protein.
77. The method of item 71, further comprising the step of isolating and sequencing a functional wild type myostatin from a said mammal not displaying muscular hyperplasia.
78. The method of item 76, further comprising comparing the complete coding sequence of the complete mutant myostatin protein with, if the coding sequence for a functional wild type myostatin from a said mammal is previously known, (1) the known sequence, or if the coding sequence for a functional wild type myostatin from a said mammal is previously unknown, (2) the sequence determined according to item 74 or item 77, to determine the location of any mutation in the mutant gene.
79. A method for determining the myostatin genotype of a mammal, wherein wild type myostatin of the mammal is substantially that of item 78, comprising:
   obtaining a sample of material containing DNA from the mammal; and
   ascertaining whether the DNA contains a said mutation determined according to item 78.
80. A method for determining the myostatin genotype of a mammal, wherein wild type myostatin of the mammal is substantially that of item 78, comprising:
   obtaining a sample of material containing mRNA from the mammal; and
   ascertaining whether the mRNA contains a said mutation determined according to item 78.
81. A primer composition useful for the detection of a nucleotide sequence encoding a myostatin comprising a first nucleic acid molecule based on a nucleotide sequence located upstream of a said mutation determined according to item 78 and a second nucleic acid molecule based on a nucleotide sequence located downstream of the mutation.
82. A probe comprising a nucleic acid molecule based on a nucleotide sequence of item 74 or item 76 and spanning a said mutation determined according to item 78.
83. A transgenic mammal having a phenotype characterized by muscular hyperplasia, said phenotype being conferred by a transgene contained in the somatic and germ cells of the mammal, the transgene encoding a myostatin protein having a dominant negative mutation.
84. The transgenic mammal of item 83 wherein the mammal is male and non-human and the transgene is located on the Y chromosome.
85. The transgenic mammal of item 83 wherein the mammal is bovine and the transgene is located to be under the control of a promoter which normally a promoter of a myosin gene.
86. A transgenic mammal having a phenotype characterized by muscular hyperplasia, said phenotype being conferred by a transgene having a sequence antisense to that encoding a myostatin protein of the mammal.
87. The transgenic mammal of item 86 wherein the mammal is bovine and the transgene is located on the Y chromosome.
88. The transgenic mammal of item 86 wherein the transgene further comprises a sequence which when transcribed obtains an mRNA having ribozyme activity.
89. A transgenic non-human mammal having a phenotype characterized by muscular hyperplasia, said phenotype being inducible and being conferred by a myostatin gene flanked by J oxP sides and a Cre transgene under the dependence of an inducible promoter.
90. A transgenic non-human male mammal having a phenotype characterized by muscular hyperplasia, said phenotype being conferred by a myostatin gene flanked by J oxP sides and a Cre transgene located on the Y chromosome.
91. A method for determining whether a sample of mammalian genetic material is capable of a conferring a phenotype characterized by muscular hyperplasia, comprising ascertaining whether the genetic material contains a nucleotide sequence encoding a protein having biological activity of myostatin, wherein the absence of said sequence indicates the presence of muscular hyperplasia in the animal.
92. A transgenic bovine having a genome lacking a gene encoding a protein having biological activity of myostatin.
93. A transgenic mouse having a genome containing a gene encoding a human protein having biological activity of myostatin or containing a gene encoding a bovine protein having biological activity of myostatin.
94. A transgenic bovine having a gene encoding a bovine protein having biological activity of myostatin and heterologous nucleotide sequence antisense to the gene.
95. A transgenic bovine of item 94, further comprising a gene encoding a nucleic acid sequence having ribozyme activity and in transcriptional association with the nucleotide sequence antisense to the gene.

### REFERENCES

Particulars of references cited above are given below. All of the listed references are incorporated herein by reference.
Barendse, W., S.M. Armitage, L.M. Kossarek, A. Shalom, B.W. Kirkpatrick, A.M. Ryan, D. Clayton, L. Li, H.L. Neibergs, N. Zhang, W.M. Grosse, J. Weiss, P. Creighton, F. McCarthy, M. Ron, A.J. Teale, R. Fries, R.A. McGraw, S.S. Moore, M. Georges, M. Soller, J.E. Womack, and D.J.S. Hetzel. 1994. A genetic linkage map of the bovine genome. Nature Genet. 6: 227-235
Bishop, M.D., S.M. Kappes, J.W. Keele, R.T. Stone, S.L.F. Sunden, G.A. Hawkins, S. Solinas Toldo, R. Fries, M.D. Grosz, J. Yoo, and C.W. Beattie. 1994. A genetic linkage map for cattle. Genetics 136: 619-639.
Boss *et al.*, United States Patent No. 4,816,397.
Cabilly *et al.* United States Patent No. 4,816,567.
Charlier, C.; Coppieters, W.; Farnir, F.; Grobet, L.; Leroy, P.; Michaux, C.; Mni, M.; Schwers, A.; Vanmanshoven, P.; Hanset, R. & Georges, M. (1995) The mh gene causing double-muscling in cattle maps to bovine chromosome 2. Mammalian Genome 6: 788-792.
Chirgwin, J.M.; Przybyla, A.E.; MacDonald, R.J.; Rutter, W.J. (1979) Isolation of biologically active ribonucleic acid from sources enriched in ribonuclease. Biochemistry 18: 5294-5299.
Cockett, N.E.; Jackson, S.P.; Shay, T.D.; Nielsen, D.; Green, R.D.; Georges, M. (1994). Chromosomal localization of the callipyge gene in sheep (Ovis aries) using bovine DNA markers. Proceedings of the National Academy of Sciences, US, 91: 3019-3023.
Cockett, N.E.; Jackson, S.P.; Shay, T.D.; Farnir, F.; Berghmans, S.; Snowder, G.; Nielsen, D.; Georges, M. (1996). Polar overdominance at the ovine callipyge locus. Science 273: 236-238.
Cole et al. (1985). Monoclonal Antibodies in Cancer Therapy. Allen R. Bliss, Inc.
Collins, F.S. 1995. Positional cloning moves from perditional to traditional. Nature Genet. 9: 347-350.
Cornelis, F.; Hashimoto, L.; Loveridge, J.; MacCarthy, A.; Buckle, V.; Julier, C.; Bell, J. (1992). Identification of a CA repeat at the TCRA locus using YACs: a general method for generating highly polymorphic markers at chosen loci. Genomics 13: 820-825.
Cottingham, R.W.; Idury, R.M.; Schäffer, A.A. (1993). Faster sequential genetic linkage computations. Am. J. Hum. Genet. 53: 252-263.
Culley, G. (1807). Observations on livestock. 4th ed., (London, G. Woodfall).
Fisher, S.R.; Beever, J.E.; Lewin, H.A. (1996). Genetic mapping of COL3AI to bovine chromosome 2. Mammalian Genome 8: 76-77.
Fuji, J.; Otsu, K.; Zorzato, F.; Deleon, S.; Khanna, V.K.; Weiler, J.E. O'Brien, P.J.; MacLennan, D.H. (1991). Identification of a mutation in the porcine ryanodine receptor associated with malignant hyperthermia. Science 253: 448-451.
Georges, M.; Andersson, L. (1996). Livestock genomics comes of age. Genome Research 6: 907-921.
Georges, M.; Nielsen, D.; Mackinnon, M.; Mishra, A.; Okimoto, R.; Pasquino, A.T.; Sargeant, L.S.; Sorensen, A.; Steele, M.R.; Zhao, X.; Womack, J.E.; Hoeschele, I. (1995). Mapping quantitative trait loci controlling milk production by exploiting progeny testing. Genetics 139: 907-920.
Gossen, M. & Bujard, H. (1992). Tight control of gene expression in mammalian cells by tetracycline-responsive promotors. Proceedings of the National Academy of Sciences, USA, 89: 5547-5551.
Grobet, L.; Royo Martin, L.J.; Poncelet, D.; Pirottin, D.; Brouwers, B.; Riquet, J.; Schoeberlein, A.; Dunner, S.; Menissier, F.; Massabanda, J.; Fries, R.; Hanset, R.; Georges, M. (1997) A deletion in the myostatin gene causes double-muscling in cattle. Nature Genetics 17: 71-74.
Gu, H.; Marth, J.D.; Orban, P.C.; Mossmann, H.; Rajewsky, K. (1994). Deletion of a DNA polymerase beta gene segment in T cells using cell type-specific gene targetting. Science 265: 103-106.
Hanset, R. and Michaux, C. (1985a). On the genetic determinism of muscular hypertrophy in the Belgian White and Blue cattle breed. I. Experimental data. Génét. Sél. Evol. 17: 359-368.
Hanset, R. and Michaux, C. (1985b). On the genetic determinism of muscular hypertrophy in the Belgian White and Blue cattle breed. II. Population data. Génét. Sél. Evol. 17: 369-386.
Hanset, R. (1991). The major gene of muscular hypetrophy in the belgian Blue Cattle Breed. In Breeding for Disease Resistance in Farm Animals, Owen, Axford, eds. C.A.B. International, pp.467-478.
Herskowitz, I. (1987). Functional inactivation of genes by dominant negative mutations. Nature 329:219-222. Hogan, B. et al., (1986). A Laboratory Manual, Cold Spring Harbor, N.Y., Cold Spring Harbor Laboratory.
Hoess, R.H.; Ziese, M.; Sternberg, N. (1982). P1 site-specific recombination: nucleotide sequence of the recombining sites. Proc.Natl.Acad.Sci.USA 79: 3398-3402.
Houbenwcyl, (1987). Methods of Organic Chemistry, ed. E. Wansch. Vol. 15 I and II. Thieme, Stuttgart.
Hudson et al. (1995) Science 270:1945-1954 with supplementary data from the Whitehead Institute/MIT Center for Genome Research, Human Genetic Mapping Project, data release 11.9 (May 1997)
Hunter, K.; Riba, L.; Schalkwyk, L.; Clark, M.; Resenchuk, S.; Beeghly, A.; Su, J.; Tinkov, F.; Lee, P.; Ramu, E.; Lehrach, H. and Housman, D. (1996). Toward the Construction of Integrated Physical and Genetic Maps of the Mouse Genome Using Interspersed Repetitive Sequence PCR (IRS\NPCR) Genomics. Genome Research 6: 290-299.
Huse et al., (1989). Science 246: 1275 -1281.
Kambadur, R.; Sharma, M.; Smith, T.P.L.; Bass, J.J. (1997). Mutations in myostatin (GDF8) in double-muscled Belgian Blue Cattle. Genome Research 7: 910-916.
Kappes, S.M.; Keele, J.W.; Stone, R.T.; McGraw, R.A.; Sonstegard, T.S.; Smith, T.P.L.; Lopez-Corrales, N.L. and Beattie, C.W. (1997). A Second-Generation Linkage Map of the Bovine Genome. Genome Research 7: 235-249.
Kennett, R. (1979). Cell fusion. Methods Enzymol. 58: 345-359.
Kohler and Milstein. (1975). Nature 256: 495-497.
Kozbor et al. (1983). Immunol. Today 4: 72.
Lathrop, M.; Lalouel, J.M. (1984). Easy calculations of lodscores and genetic risk on small computers. American Journal of Human Genetics 36: 460-465.
Lenstra, J.A.; van Boxtel, J.A.F.; Zwaagstra, K.A.; Schwerin, M. (1993). Short interspersed nuclear element (SINE) sequences of the Bovidae. Animal Genetics 24: 33-39.
Libert, F.; Lefort, A.; Okimoto, R.; Georges, M. (1993) Construction of a bovine genomic library of large yeast artificial chromosome clones. Genomics 18: 270-276.
Lopez, A.R.; Cook, J.; Deininger, P.L.; Derynck, R. (1992). Dominant negative mutants of trnasforming growth factor-beta1 inhibit the secretation of different transforming growth factor beta isoforms. Molecular and Cellular biology 12(4): 1674-1679.
Lyons, A.L.; Laughlin, T.F.; Copeland, N.G.; Jenkins, N.A.; Womack, J.E.; O'Brien, S.J. (1996). Comparative Anchor tagged Sequences for Integrative mapping of Mammalian Genomes. Nature Genetics 15: 47-56.
McPherron, A.C.; Lee, S.-J. (1996). The transforming growth factor β superfamily. In Growth Factors and Cytokines in Health and Disease, Volume 1B, pages 357-393. JAI press Inc.
McPherron, A.C.; Lawler, A.M.; Lee, S.-J. (1997). Regulation of skeletal muscle mass in mice by a new TGFβ superfamily member. Nature 387: 83-90.
Menissier, F. (1982). Present state of knowledge about the genetic determination of muscular hypertrophy or the double muscled trait in cattle. in Current Topics in Veterinary Medicine and Animal Science, vol. 16: Muscle hypertrophy of genetic origin and its use to improve beef production, pp. 387-428. Ed. King and Menissier, Martinus Nijhoff.
Merrifield, (1964]. J. Am. Chem. Assoc. 85: 2149-2154.
McCafferty et al., (1990). Nature 348: 552-554.
Mirski, S. and Cole, S. P. C. (1989). Antigens associated with multidrug resistance in H69AR, a small cell lung cancer cell line. Cancer Res. 49: 5719-5724.
Morrison et al., (1985). Proceedings of the National Academy of Sciences, USA, 81: 6851.
O'Brien, S.J.; Womack, J.E.; Lyons, L.A.; Moore, K.J.; Jenkins, N.A.; Copeland, N.G. (1993). Anchored reference loci for comparative genome mapping in mammals. Nature Genetics 3: 103-112.
Old, R.W. and Primrose, S.B., In: Principles of Gene Manipulation. An Introduction to Genetic Engineering, 4th ed. Oxford University Press. 63-66.
Pell, J.M.; Flint, D.J.; (1997). In: Milk Composition, Production and Biotechnology, Ed. Welch et al., Chapter 19.
Sambrook, J., Fritsch E.F. and Maniatis, T. (1989). Molecular Cloning: A Laboratory Manual. Cold Spring Harbor Lab Press, Cold Spring Harbor, New York.
Shockett, P.E.; Schatz, D.G. (1996). Diverse strategies for tetracycline-regulated inducible gene expression. Proceedings of the National Academy of Sciences, USA, 93: 5173-5176.
Solinas-Toldo, S.; Lengauer, C; Fries, R. (1995). Comparative genome map of man and cattle. Genomics 27: 489-496.
Staerz & Bevan (1986a). Proceedings of the National Academy of Sciences, USA, 83: 1453.
Staerz & Bevan (1986b). Immunology Today 7: 241.
Stewart, A.J., Canitrot, Y., Baracchini, E., Dean, N.M., Deeley, R.G., and Cole, S.P.C. (1996). Reduction of Expression of the multidrug resistance protein (MRP) in human tumor cells by antisense phophorothioate oligonucleotides. Biochem. Pharamcol. 51: 461-469.
Takeda et al., (1985). Nature 314: 452.
Tanaguchi *et al.*, European Patent Publication EP171496.
Teng, et al., (1982) Meth. Enzymol. 92: 3-16.
Varga, L.; Szabo, G.; Darvasi, A.; Müller, G.; Sass, M.; Soller, M. (1997). Inheritance and mapping of compact (Cmpt), a new mutation causing hypermuscularity in mice. Genetics, in the press.
Walter, M.A.; Spillett, D.J.; Thomas, P.; Weissenbach, J.; Goodfellow, P.N. (1994). A method for constructing radiation hybrid maps of whole genomes. Nature Genetics 7:22-28. Ward et al., (1989). Nature 341: 544-546.

## Claims

1. A diagnostic kit, for determining the genotype of a sample of mammalian genetic material, the kit comprising:
a pair of primers, wherein a first of the primers includes a nucleotide sequence of sufficient length and sufficiently complementary to a mutation of SEQ ID NO: 1 and which binds under high stringency conditions to a nucleic acid sequence containing said mutation, the mutation being selected from the group of mutations resulting from:
(a) deletion of 11 nucleotides beginning at nucleotide 821 of the coding portion of SEQ ID NO:1;
(b) deletion of 7 nucleotides beginning at nucleotide 419 of the coding sequence and insertion of the sequence AAGCATACAA in place thereof;
(c) deletion of nucleotide 610 of the coding sequence and insertion of T in place thereof;
(d) deletion of nucleotide 676 of the coding sequence and insertion of T in place thereof; and
(e) combinations of mutations (a) to (d).

2. The diagnostic kit of claim 1 wherein a second of the pair of primers is located entirely upstream or entirely downstream of the selected mutation or mutations.

3. The diagnostic kit of claim 2 wherein a first said primer spans mutation (a) and further comprising a third primer which is sufficiently complementary to the nucleotide sequence identified as SEQ ID NO: 11 to prime amplification of a nucleic acid molecule containing SEQ ID NO:11.

4. The diagnostic kit of claim 2 wherein a first said primer is sufficiently complementary to the inserted sequence of mutation (b) to prime amplification of a nucleic acid molecule containing mutation (b) and further comprising a third primer which is sufficiently complementary to the sequence corresponding to the 7 nucleotide deletion of mutation (b) to prime amplification of a nucleic acid molecule containing the 7 nucleotide deletion of mutation (b).

5. The diagnostic kit of claim 2 wherein a first said primer spans mutation (c) and further comprising a third primer which is sufficiently complementary to the sequence spanning the corresponding region lacking mutation (c) to prime amplification of a nucleic acid molecule lacking mutation (c).

6. The diagnostic kit of claim 2 wherein a first said primer spans mutation (d) and further comprising a third primer which is sufficiently complementary to the sequence spanning the corresponding region lacking mutation (d) to prime amplification of a nucleic acid molecule lacking mutation (d).

7. A method for determining the presence of muscular hyperplasia in a bovine animal, the method comprising:
ascertaining whether DNA having a mutation as defined in claim 1 is present in a sample of material containing DNA from said animal using primers as defined in claim 1 or a probe of sufficient length and sufficiently complementary to a nucleic acid molecule containing said mutation and which binds under high stringency conditions with a nucleic acid sequence containing said mutation; and ascertaining whether DNA having a nucleotide sequence encoding a protein having biological activity of myostatin is present, wherein the absence of DNA having said nucleotide sequence and presence of said mutation indicates the presence of muscular hyperplasia in the animal.

8. The method of claim 7 wherein the animal is a breed selected from Belgian Blue, Asturiana, Parthenaise and Rubia Gallega.

## Patentansprüche

1. Diagnose-Kit zur Bestimmung des Genotyps einer Probe von genetischem Material eines Säugers, wobei der Kit umfasst:
ein Primer-Paar, wobei ein erster Primer eine Nucleotidsequenz umfasst, die ausreichender Länge und ausreichend komplementär zu einer Mutation von SEQ ID NO:1 ist, und der unter hohen Stringenz-Bedingungen an eine Nucleinsäuresequenz bindet, die die Mutation enthält, wobei die Mutation ausgewählt ist aus der Gruppe von Mutationen, die resultieren aus:
(a) Deletion von 11 Nucleotiden beginnend bei Nucleotid 821 des codierenden Teils von SEQ ID NO:1;
(b) Deletion von 7 Nucleotiden beginnend bei Nucleotid 419 der codierenden Sequenz und Insertion der Sequenz AAGCATACAA anstelle davon;
(c) Deletion von Nucleotid 610 der codierenden Sequenz und Insertion von T anstelle davon;
(d) Deletion von Nucleotid 676 der codierenden Sequenz und Insertion von T anstelle davon; und
(e) Kombinationen der Mutationen (a) bis (d).

2. Diagnose-Kit gemäß Anspruch 1, wobei ein zweiter Primer des Primer-Paares vollständig stromaufwärts oder vollständig stromabwärts der ausgewählten Mutation oder Mutationen lokalisiert ist.

3. Diagnose-Kit gemäß Anspruch 2, wobei sich ein erster Primer über Mutation (a) erstreckt, und des Weiteren einen dritten Primer umfassend, der ausreichend komplementär zu der mit SEQ ID NO:11 bezeichneten Nucleotidsequenz ist, um die Amplifikation eines Nucleinsäuremoleküls, das SEQ ID NO:11 enthält, einzuleiten.

4. Diagnose-Kit gemäß Anspruch 2, wobei ein erster Primer ausreichend komplementär zu der inserierten Sequenz von Mutation (b) ist, um die Amplifikation eines Nucleinsäuremoleküls, das Mutation (b) enthält, einzuleiten, und des Weiteren einen dritten Primer umfassend, der ausreichend komplementär zu der Sequenz ist, die der Deletion von 7 Nucleotiden von Mutation (b) entspricht, um die Amplifikation eines Nucleinsäuremoleküls, das die Deletion der 7 Nucleotide von Mutation (b) enthält, einzuleiten.

5. Diagnose-Kit gemäß Anspruch 2, wobei sich ein erster Primer über Mutation (c) erstreckt, und des Weiteren einen dritten Primer umfassend, der ausreichend komplementär zu der Sequenz ist, die sich über die entsprechende Region erstreckt, der Mutation (c) fehlt, um die Amplifikation eines Nucleinsäuremoleküls, dem Mutation (c) fehlt, einzuleiten.

6. Diagnose-Kit gemäß Anspruch 2, wobei sich ein erster Primer über Mutation (d) erstreckt, und des Weiteren einen dritten Primer umfassend, der ausreichend komplementär zu der Sequenz ist, die sich über die entsprechende Region erstreckt, der Mutation (d) fehlt, um die Amplifikation eines Nucleinsäuremoleküls, dem Mutation (d) fehlt, einzuleiten.

7. Verfahren zur Bestimmung des Vorliegens von muskulärer Hyperplasie bei einem Rind, wobei das Verfahren umfasst:
Feststellen, ob DNA, die eine wie in Anspruch 1 definierte Mutation aufweist, in einer Materialprobe vorliegt, die DNA des Tieres enthält, unter Verwendung von wie in Anspruch 1 definierten Primern oder einer Sonde, die ausreichender Länge und ausreichend komplementär zu einem Nucleinsäuremolekül ist, das die Mutation enthält und unter hohen Stringenz-Bedingungen an eine Nucleinsäuresequenz, die die Mutation enthält, bindet; und Feststellen, ob DNA, die eine Nucleotidsequenz aufweist, die ein Protein mit biologischer Aktivität von Myostatin codiert, vorliegt, wobei das Fehlen von DNA, die die Nucleotidsequenz aufweist, und das Vorliegen der Mutation darauf hinweist, dass bei dem Tier muskuläre Hyperplasie vorliegt.

8. Verfahren gemäß Anspruch 7, wobei das Tier eine Rasse ausgewählt aus Weißblaue Belgier (Belgian Blue), Asturiana, Parthenaise und Gallicisches Blondvieh (Rubia Gallega) ist.

## Revendications

1. Kit de diagnostic, pour la détermination du génotype d'un échantillon de matériel génétique de mammifère, le kit comprenant :
une paire d'amorces, dans laquelle une première amorce comprend une séquence nucléotidique de longueur suffisante et suffisamment complémentaire à une mutation de SEQ ID NO : 1 et qui se lie dans des conditions de stringence élevée à une séquence d'acide nucléique contenant ladite mutation, la mutation étant choisie dans le groupe de mutations résultant de :
(a) la délétion de 11 nucléotides débutant au nucléotide 821 de la partie codante de SEQ ID NO : 1 ;
(b) la délétion de 7 nucléotides débutant au nucléotide 419 de la séquence codante et l'insertion de la séquence AAGCATACAA à sa place ;
(c) la délétion du nucléotide 610 de la séquence codante et l'insertion de T à sa place ;
(d) la délétion du nucléotide 676 de la séquence codante et l'insertion de T à sa place ;
(e) des combinaisons des mutations (a) à (d).

2. Kit de diagnostic selon la revendication 1, dans lequel une seconde amorce de la paire d'amorces est localisée entièrement en amont ou entièrement en aval de la mutation ou des mutations choisies.

3. Kit de diagnostic selon la revendication 2, dans lequel une première dite amorce couvre la mutation (a) et comprenant en outre une troisième amorce qui est suffisamment complémentaire à la séquence nucléotidique identifiée comme SEQ ID NO : 11 pour amorcer l'amplification d'une molécule d'acide nucléique contenant SEQ ID NO : 11.

4. Kit de diagnostic selon la revendication 2, dans lequel une première dite amorce est suffisamment complémentaire à la séquence insérée de la mutation (b) pour amorcer l'amplification d'une molécule d'acide nucléique contenant la mutation (b) et comprenant en outre une troisième amorce qui est suffisamment complémentaire à la séquence correspondant à la délétion de 7 nucléotides de la mutation (b) pour amorcer l'amplification d'une molécule d'acide nucléique contenant la délétion de 7 nucléotides de la mutation (b).

5. Kit de diagnostic selon la revendication 2, dans lequel une première dite amorce couvre la mutation (c) et comprenant en outre une troisième amorce qui est suffisamment complémentaire à la séquence couvrant la région correspondante à laquelle il manque la mutation (c) pour amorcer l'amplification d'une molécule d'acide nucléique à laquelle il manque la mutation (c).

6. Kit de diagnostic selon la revendication 2, dans lequel une première dite amorce couvre la mutation (d) et comprenant en outre une troisième amorce qui est suffisamment complémentaire à la séquence couvrant la région correspondante à laquelle il manque la mutation (d) pour amorcer l'amplification d'une molécule d'acide nucléique à laquelle il manque la mutation (d).

7. Méthode de détermination de la présence d'une hyperplasie musculaire chez un animal bovin, la méthode comprenant :
la détermination de la présence d'ADN comportant une mutation telle que définie dans la revendication 1 dans un échantillon de matériel contenant de l'ADN provenant dudit animal en utilisant des amorces telles que définies dans la revendication 1 ou une sonde de longueur suffisante et suffisamment complémentaire à une molécule d'acide nucléique contenant ladite mutation et qui se lie dans des conditions de stringence élevée à une séquence d'acide nucléique contenant ladite mutation ; et la détermination de la présence d'ADN ayant une séquence nucléotidique codant pour une protéine possédant une activité biologique de myostatine, dans laquelle l'absence d'ADN ayant ladite séquence nucléotidique et la présence de ladite mutation indique la présence d'une hyperplasie musculaire chez l'animal.

8. Méthode selon la revendication 7, dans laquelle l'animal est une race choisie parmi Belgian Blue, Asturiana, Parthenaise et Rubia Gallega.
